# EUROPEAN PATENT SPECIFICATION

(11) **EP 2 215 266 B1**
(45) Date of publication and mention of the grant of the patent: **14.01.2015**
(21) Application number: 08849756.5
(22) Date of filing: 13.11.2008
(51) Int. Cl.: C12Q 1/68

(54) **DIAGNOSTIC BIOMARKERS OF DIABETES**
DIAGNOSTISCHE BIOMARKER FÜR DIABETES
BIOMARQUEURS DIAGNOSTIQUES DU DIABÈTE

(30) Priority: 13.11.2007 US 987540 P
(43) Date of publication of application: 11.08.2010
(60) Divisional application: 13151356.6
(73) Proprietor: Janssen Diagnostics, LLC, Raritan, NJ 08869 (US)
(72) Inventor: PALMA, John, F., Carlsbad, CA 92009 (US); BACKUS, John, W., Ontario,NY 14519 (US); ZHANG, Yi, San Diego,CA 92127 (US); WANG, Yixin, Basking Ridge, NJ 07920 (US); YU, Jack, X., San Diego,CA 92128 (US); VENER, Tatiana, Stirling, NJ 07980 (US); DERECHO, Carlo, Lakehurst, NJ 08733 (US); LEE, Dong-Ho, Kyunggi-Do, Korea 445-922 (KR)
(74) Representative: Goodfellow, Hugh Robin
(86) International application number: PCT/US2008/083424
(87) International publication number: WO 2009/064901

(56) References cited:
- EP-A- 1 538 219
- WO-A-02/22886
- FR-A- 2 910 025
- MA ET AL: "Identifying leukocyte gene expression patterns associated with plasma lipid levels in human subjects" ATHEROSCLEROSIS, ELSEVIER IRELAND LTD, IE, vol. 191, no. 1, 13 February 2007 (2007-02-13), pages 63-72, XP005886383 ISSN: 0021-9150
- GLASGOW S C ET AL: "Unfavourable expression of pharmacologic markers in mucinous colorectal cancer" BRITISH JOURNAL OF CANCER, vol. 92, no. 2, 31 January 2005 (2005-01-31), pages 259-264, XP002523552 ISSN: 0007-0920
- KAIZER ELLEN C ET AL: "Gene expression in peripheral blood mononuclear cells from children with diabetes." THE JOURNAL OF CLINICAL ENDOCRINOLOGY AND METABOLISM SEP 2007, vol. 92, no. 9, September 2007 (2007-09), pages 3705-3711, XP002523553 ISSN: 0021-972X
- SU MIN-CHENG ET AL: "CD24 expression is a prognostic factor in intrahepatic cholangiocarcinoma." CANCER LETTERS 8 APR 2006, vol. 235, no. 1, 8 April 2006 (2006-04-08), pages 34-39, XP002523554 ISSN: 0304-3835
- Roche Applied Science: "Lightcycler Faststart DNA master Sybr Geen I", , January 2005 (2005-01), Retrieved from the Internet: URL:https://www.roche-applied-science.com/ PROD_INF/index.jsp?id=iforu&iforu_page=sea rch&catalogNumber=03003230001&instruction_ version=Version+09 [retrieved on 2013-02-20]

## Description

### FIELD OF THE APPLICATION

The application relates to the field of medical diagnostics and describes methods and kits for the point-of-care diagnosis of a diabetic disease state in a patient.

### BACKGROUND OF THE INVENTION

Diabetes is a group of diseases marked by high levels of blood glucose resulting from defects in insulin production, insulin action, or both. Left untreated, it can cause many serious short term complications including symptoms of hypoglycemia, ketoacidosis, or nonketotic hyperosmolar coma. In the long term, diabetes is known to contribute to an increased risk of arteriosclerosis, chronic renal failure, retinal damage (including blindness), nerve damage and microvascular damage.

The spreading epidemic of diabetes in the developing world is predicted to have a profound impact on the healthcare system in the United States. A recent study by the Center for Disease Control and Prevention indicates the incidence of new diabetes cases in the U.S. nearly doubled in the last 10 years. As of 2007, at least 57 million people in the United States have pre-diabetes. Coupled with the nearly 24 million who already have diabetes, this places more than 25% of the U.S. population at risk for further complications from this disease. According to the American Diabetes Association, the estimated cost of diabetes in the United States in 2007 amounted to $174 billion with direct medical costs approaching $116 billion.

WO02/22886 relates to a gene expression pattern which is allegedly different in adipose cells of individuals who have Type 2 Diabetes Mellitus from the cells of individuals who do not have the disease.

EP1538219 discloses methods which are alleged to be able to be used to diagnose or screen for Diabetes Mellitus type 1 (DM1) in a subject by assaying for a marker for DM1.

Ma, 2007, Atherosclerosis 191:63-72 discloses the results of an investigation into the relationship between plasma lipids and leukocytes.

Although the etiology of diabetes appears to be multi-factorial in nature, increasing experimental evidence suggests the onset of obesity, especially abdominal obesity, disrupts immune and metabolic homeostasis and ultimately leads to a broad inflammatory response. The production of inflammatory cytokines in the adipose tissue, such as TNF alpha, then deregulates the immune response and a cell's ability to respond to insulin. Detection of an alteration in the transcriptional profiles of circulating immune cells, such as monocytes and macrophages, therefore provides a convenient avenue to diagnose the disease and monitor its progression before even the more overt signs of glucose intolerance become apparent.

For the forgoing reasons, there is an unmet need for rapid and accurate diagnostic assays for the diagnosis and monitoring of patients at risk of developing diabetes. In particular, there is an unmet need for diagnostic assays in a kit format that can be readily used at a point-of-care facility for the routine screening of patients for early onset diabetes.

### SUMMARY OF THE APPLICATION

Methods are described for the determination of gene signature expression profiles that are diagnostic of pre-diabetic and diabetic disease states. The disclosure further pertains to diagnostic kits comprising reagents for the rapid measurement of gene signature expression profiles in a patient's blood sample. The kit format is cost-effective and convenient for use at a point-of-care facility.

In one embodiment, a method is described for the diagnosis of Diabetes Mellitus in a patient, the method comprising the steps of (a) providing a test sample taken from a patient, (b) measuring the gene expression profile of a gene signature comprising
the TOP1, CD24 and STAP1 genes, (c) comparing the gene expression profile with a diagnostic gene expression profile of the gene signature, (d) determining a diabetic disease state in the patient based at least in part upon a substantial match between the gene expression profile and the diagnostic gene expression profile and (e) displaying the determination to a medical professional.

The determining step can be executed by a computer system running one or more algorithms selected from the group of Linear combination of gene expression signals, Linear regression model, Logistic regression model, Linear discrimination analysis (LDA) model, The nearest neighbor model and the Prediction Analysis of Microarrays (PAM). The determining step can also include analysis of the patient's metabolic disease profile.

In one embodiment, the gene signature can include the TOP1, CD24 and STAP1 genes and one or more genes selected from the genes listed in TABLES 1 or 6.

The patient can have a normal BMI. The diabetic disease state can be a pre-diabetic disease state or a Type 2 Diabetes disease state.

The test sample can be a blood sample or a test sample containing PBMCs or CD11c⁺ or CD11b⁺ or Emr⁺ or [CD11b⁺ CD11c⁺] or [Emr⁺CD11b⁺] or [Emr⁺ CD 11c⁺] or [Emr⁺ CD11b⁺ CD11c⁺] cells or CD14⁺ monocytes.

The measuring can involve real-time PCR, an immunochemical assay or a specific oligonucleotide hybridization.

In another embodiment of the disclosure, a method is described for the diagnosis of Diabetes Mellitus in a patient, the method comprising the steps of (a) providing a test sample taken from a patient, (b) measuring the gene expression profile of a gene signature comprising a gene selected from the group of TULP4, AA741300, ESCO1, EIF5B, ACTR2, WNK1, COCH, SON, TPR and NOG genes, (c) comparing the gene expression profile with a diagnostic gene expression profile of the gene signature, (d) determining a diabetic disease state in the patient based at least in part upon a substantial match between the gene expression profile and the diagnostic gene expression profile and (e) displaying the determination to a medical professional.

The determining step can be executed by a computer system running one or more algorithms selected from the group of Linear combination of gene expression signals, Linear regression model, Logistic regression model, Linear discrimination analysis (LDA) model, The nearest neighbor model and the Prediction Analysis of Microarrays (PAM). The determining step can also include analysis of the patient's metabolic disease profile.

The gene signature can include any two genes or any three genes selected from the group of TULP4, AA741300, ESCO1, EIF5B, ACTR2, WNK1, COCH, SON, TPR and NOG genes. In one aspect, the gene signature includes the TULP4, AA741300, ESCO1, EIF5B, ACTR2, WNK1, COCH, SON, TPR and NOG genes. In one aspect, the gene signature includes the TOP1, CD24 and STAP1 genes in addition to at least one gene selected from the group of the TULP4, AA741300, ESCO1, EIF5B, ACTR2, WNK1, COCH, SON, TPR and NOG genes. In another aspect, the gene signature includes one or more genes selected from the group of TULP4, AA741300, ESCO1, EIF5B, ACTR2, WNK1, COCH, SON, TPR and NOG genes and one or more genes selected from the genes listed in TABLES 1 or 6.

The patient can have a normal BMI. The diabetic disease state can be a pre-diabetic disease state or a Type 2 Diabetes disease state.

The test sample can be a blood sample or a test sample containing PBMCs or CD11c⁺ or CD11b⁺ or Emr⁺ or [CD11b⁺ CD11c⁺] or [Emr⁺CD11b⁺] or [Emr⁺CD11c⁺] or [Emr⁺CD11b⁺CD11c⁺] cells or CD14⁺ monocytes.

The measuring can involve real-time PCR, an immunochemical assay or a specific oligonucleotide hybridization.

In one embodiment, a method is described for the diagnosis of Diabetes Mellitus in a patient, the method comprising the steps of (a) providing a test sample taken from a patient, (b) measuring the gene expression profile of a gene signature comprising the TCF7L2 and CLC genes, (c) comparing the gene expression profile with a diagnostic gene expression profile of the gene signature, (d) determining a diabetic disease state in the patient based at least in part upon a substantial match between the gene expression profile and the diagnostic gene expression profile and (e) displaying the determination to a medical professional.

The determining step can be executed by a computer system running one or more algorithms selected from the group of Linear combination of gene expression signals, Linear regression model, Logistic regression model, Linear discrimination analysis (LDA) model, The nearest neighbor model and the Prediction Analysis of Microarrays (PAM). The determining step can also include analysis of the patient's metabolic disease profile.

The gene signature can include either the TCF7L2 or CLC gene. In one aspect, the gene signature includes one or more variants of the TC7L2 or CLC gene. In one aspect, the gene signature includes either the TCF7L2 or CLC gene and one or more genes selected from the genes listed in TABLES 1 or 6.

The patient can have a normal BMI. The diabetic disease state can be a pre-diabetic disease state or a Type 2 Diabetes disease state.

The test sample can be a blood sample or a test sample containing PBMCs or CD11c⁺ or CD11b⁺ or Emr⁺ or [CD11b⁺ CD11c⁺] or [Emr⁺CD11b⁺] or [Emr⁺CD11c⁺] or [Emr⁺CD11b⁺CD11c⁺] cells or CD14⁺ monocytes.

The measuring can involve real-time PCR, an immunochemical assay or a specific oligonucleotide hybridization.

In one embodiment, a method is described for diagnosing a change in the diabetic disease state of a patient comprising the steps of (a) providing a first test sample taken from a patient at a first time point, (b) measuring a first expression profile of a gene signature comprising the TOP1, CD24 and STAP1 genes in the first test sample, (c) providing a second test sample taken from the patient at a second time point, (d) measuring a second expression profile of the gene signature in the second test sample, (e) comparing the first expression profile with the second expression profile, (f) determining, a change in the diabetic disease state in the patient based at least in part upon a substantial difference between the first gene expression profile and the second gene expression profile, and (g) displaying the determination to a medical professional.

In one aspect, the determining step is executed by a computer system running one or more algorithms selected from the group of Linear combination of gene expression signals, Linear regression model, Logistic regression model, Linear discrimination analysis (LDA) model, The nearest neighbor model and the Prediction Analysis of Microarrays (PAM). In another aspect, the determining step also includes an analysis of the patient's metabolic disease profile.

In another aspect, the gene signature includes a gene selected from the group of the TOP1, CD24 and STAP1 genes and one or more genes selected from the genes listed in TABLES 1 or 6.

In one aspect, the time period between the first time point and the second time point is from 0 to 2 years or from ¼ to 2 years or from ¼ to 2 years or from 2 to 5 years, or from 5 to 10 years or more.

A change in diabetic disease state can be indicative of a progression toward a pre-diabetic disease state or a Type II Diabetes disease state. In one aspect, the patient at the first time point gas a normal BMI.

The first and second test sample can be blood samples. In one aspect, the first and second test sample can a test sample containing PBMCs or CD11c⁺ or CD 11b⁺ or Emr⁺ or [CD11b⁺ CD11c⁺] or [Emr⁺CD11b⁺] or [Emr⁺CD11c⁺] or [Emr⁺CD11b⁺ CD11c⁺] cells or CD14⁺ monocytes.

The measuring can involve real-time PCR, an immunochemical assay or a specific oligonucleotide hybridization.

In one embodiment of the disclosure, a method is described for diagnosing a change in the diabetic disease state of a patient comprising the steps of (a) providing a first test sample taken from a patient at a first time point, (b) measuring a first expression profile of a gene signature comprising a gene selected from the group of the TULP4, AA741300, ESCO1, EIF5B, ACTR2, WNK1, COCH, SON, TPR and NOG genes in the first test sample, (c) providing a second test sample taken from the patient at a second time point, (d) measuring a second expression profile of the gene signature in the second test sample, (e) comparing the first expression profile with the second expression profile, (f) determining a change in the diabetic disease state in the patient based at least in part upon a substantial difference between the first gene expression profile and the second gene expression profile, and (g) displaying the determination to a medical professional.

In one aspect, the determining step is executed by a computer system running one or more algorithms selected from the group of Linear combination of gene expression signals, Linear regression model, Logistic regression model, Linear discrimination analysis (LDA) model, The nearest neighbor model and the Prediction Analysis of Micro arrays (PAM). In another aspect, the determining step also includes an analysis of the patient's metabolic disease profile.

The gene signature can include any two genes selected from the group of the TULP4, AA741300, ESCO1, EIF5B, ACTR2, WNK1, COCH, SON, TPR and NOG genes. In another aspect, the gene signature includes any three genes selected from the group of TULP4, AA741300, ESCO1, EIF5B, ACTR2, WNK1, COCH, SON, TPR and NOG genes. In one aspect, the gene signature includes the TULP4, AA741300, ESCO1, EIF5B, ACTR2, WNK1, COCH, SON, TPR and NOG genes. In another aspect, the gene signature includes one or more genes selected from the group of the TULP4, AA741300, ESCO1, EIF5B, ACTR2, WNK1, COCH, SON, TPR and NOG genes and one or more genes selected from the genes listed in TABLES 1 or 6.

In one aspect, the time period between the first time point and the second time point is from 0 to 2 years or from ¼ to 2 years or from ½ to 2 years or from 2 to 5 years, or from 5 to 10 years or more.

A change in diabetic disease state can be indicative of a progression toward a pre-diabetic disease state or a Type II Diabetes disease state. In one aspect, the patient at the first time point gas a normal BMI.

The first and second test sample can be blood samples. In one aspect, the first and second test sample can a test sample containing PBMCs or CD11c⁺ or CD11b⁺ or Emr⁺ or [CD11b⁺CD11c⁺] or [Emr⁺CD11b⁺] or [Emr⁺CD11c⁺] or [Emr⁺CD11b⁺ CD11c⁺] cells or CD14⁺ monocytes.

The measuring can involve real-time PCR, an immunochemical assay or a specific oligonucleotide hybridization.

In another embodiment, a kit is described for assessing a patient's susceptibility to Diabetes in which the assessment is made with a test apparatus. The kit includes (a) reagents for collecting a test sample from a patient; and (b) reagents for measuring the expression profile of a gene signature comprising the TCF7L2 and CLC genes or variants thereof in a patient's test sample.

Reagents in step (a) and (b) are sufficient for a plurality of tests. Reagents for collecting a test sample from a patient can be packaged in sterile containers.

The gene signature can include one or more of the genes selected from the group of TCF7L2 and CLC genes and one or more genes selected from the list of genes of TABLES 1 or 6.

The test sample can be a blood sample.

The kit can also include reagents for the isolation of PBMCs or reagents for the iso lation of CD11c⁺ or CD11b⁺ or Emr⁺ or [CD11b⁺CD11c⁺] or [Emr⁺CD11b⁺] or [Emr⁺ CD11c⁺] or [Emr⁺CD11b⁺CD11c⁺] cells or reagents for the isolation of CD14⁺ monocytes. The reagents for measuring the expression profile of a gene signature can be real-time PCR reagents, immunochemical assay reagents or for specific oligonucleotides hybridization.

In another embodiment, a kit is described for assessing a patient's susceptibility to Diabetes in which the assessment is made with a test apparatus. The kit includes (a) reagents for collecting a test sample from a patient; and (b) reagents for measuring the expression profile of a gene signature comprising the TOP1, CD24 and STAP1 genes in a patient's test sample.

In another aspect, the gene signature includes the TOP1, CD24 and STAP1 genes and one or more genes selected from the list of genes of TABLES 1 or 6.

Reagents in step (a) and (b) are sufficient for a plurality of tests. Reagents for collecting a test sample from a patient can be packaged in sterile containers.

The test sample can be a blood sample.

The kit can also include reagents for the isolation of PBMCs or reagents for the isolation of CD11c+ or CD11b+ or Emr+ or [CD11b+ CD11c+] or [Emr+ CD11b+] or [Emr+ CD11c+] or [Emr+ CD11b+CD11c+] cells or reagents for the isolation of CD14+ monocytes. The reagents for measuring the expression profile of a gene signature can be real-time PCR reagents as defined in the claims, or immunochemical assay reagents as defined in the claims.

In another embodiment of the disclosure, a kit is described for assessing a patient's susceptibility to Diabetes in which the assessment is made with a test apparatus. The kit includes (a) reagents for collecting a test sample from a patient; and (b) reagents for measuring the expression profile of a gene signature a gene or variant thereof selected from the group of TULP4, AA741300, ESCO1, EIF5B, ACTR2, WNK1, COCH, SON, TPR and NOG genes in a patient's test sample.

In one aspect, the gene signature comprises one or more genes selected from the group of TULP4, AA741300, ESCO1, EIF5B, ACTR2, WNK1, COCH, SON, TPR and NOG genes. In one aspect, the gene signature comprises two or more genes selected from the group of TULP4, AA741300, ESCO1, EIF5B, ACTR2, WNK1, COCH, SON, TPR and NOG genes. In one aspect, the gene signature comprises three or more genes selected from the group of TULP4, AA741300, ESCO1, EIF5B, ACTR2, WNK1, COCH, SON, TPR and NOG genes.

Reagents in step (a) and (b) are sufficient for a plurality of tests. Reagents for collecting a test sample from a patient can be packaged in sterile containers.

The gene signature can also include one or more genes selected from the group of TULP4, AA741300, ESCO1, EIF5B, ACTR2, WNK1, COCH, SON, TPR and NOG genes and one or more genes selected from the list of genes of TABLES 1 or 6.

The test sample can be a blood sample.

The kit can also include reagents for the isolation of PBMCs or reagents for the isolation of CD11c⁺ or CD11b⁺ or Emr⁺ or [CD11b⁺CD11c⁺] or [Emr⁺CD11b⁺] or [Emr⁺CD11c⁺] or [Emr⁺CD 11b⁺CD 11c⁺] cells or reagents for the isolation of CD 14⁺ monocytes. The reagents for measuring the expression profile of a gene signature can be real-time PCR reagents, immunochemical assay reagents or for specific oligonucleotides hybridization.

It should be understood that this application is not limited to the embodiments disclosed in this Summary, and it is intended to cover modifications and variations that are within the scope of the claims.

The previously described embodiments have many advantages, including novel gene signatures for the early diagnosis of a pre-diabetic disease state and the monitoring of patients who are at risk of developing diabetes or who have already acquired the disease. The disclosure also describes kits with reagents and instructions for the cost-effective and rapid testing of blood samples by medical personnel at a point-of-care facility.

### BRIEF DESCRIPTION OF THE DRAWING

FIG. 1 depicts a ROC Curve Analysis of GLC Gene compared to OGTT in accordance with a first embodiment;
FIG. 2A depicts a ROC Curve Analysis of TCF7L2 set 1 compared to OGTT according to a second embodiment;
FIG 2B depicts a ROC Curve Analysis of TCF7L2 set 1 compared to compared to OGTT vs. FPG according to a third embodiment;
FIG. 3 shows a ROC Curve Analysis of CDKN1C gene according to a fourth embodiment;
FIG. 4A shows a ROC analysis of the 3-gene signature compared to OGTT according to a fifth embodiment;
FIG. 4B depicts a ROC analysis of the 3-gene signature compared to FPG vs. OGTT according to a sixth embodiment;
FIG. 4C depicts bar chart of the mean expression of the 3-gene signature according to a seventh embodiment;
FIG. 5A shows a ROC analysis of the 10-gene signature compared to OGTT according to an eighth embodiment;
FIG. 5B shows a ROC analysis of the 10-gene signature compared to FPG vs. OGTT according to a ninth embodiment; and
FIG. 5C depicts bar chart of the mean expression of the 10-gene signature according to a tenth embodiment

### DETAILED DESCRIPTION

Unless defined otherwise, all technical and scientific terms used herein have the same meaning as is commonly understood by one of skill in the art. The following definitions are provided to help interpret the disclosure and claims of this application. In the event a definition in this section is not consistent with definitions elsewhere, the definition set forth in this section will control.

Furthermore, the practice of the invention employs, unless otherwise indicated, conventional molecular biological and immunological techniques within the skill of the art. Such techniques are well known to the skilled worker, and are explained fully in the literature. See, e.g., Colignan, Dunn, Ploegh, Speicher and Wingfield "Current protocols in Protein Science" (1999-2008) Volume I and II, including all supplements (John Wiley & Sons Inc.); and Bailey, J. E. and Ollis, D. F., Biochemical Engineering Fundamentals, McGraw-Hill Book Company, NY, 1986; Ausubel, et al., ed., Current Protocols in Molecular Biology, John Wiley & Sons, Inc., NY, N.Y. (1987-2008), including all supplements; Sambrook, et al., Molecular Cloning: A Laboratory Manual, 2nd Edition, Cold Spring Harbor, N.Y. (1989); and Harlow and Lane, Antibodies, a Laboratory Manual, Cold Spring Harbor, N.Y. (1989). ROC analysis is reviewed in "An Introduction to ROC Analysis" by Tom Fawcett, Pattern Recognition Letters 27 (2006) 861-874.

As used herein, "Diabetes Mellitus" refers to any disease characterized by a high concentration of blood glucose (hyperglycemia). Diabetes mellitus is diagnosed by demonstrating any one of the following: a fasting plasma glucose level at or above 126 mg/dL (7.0 mmol/l) or a plasma glucose at or above 200 mg/dL (11.1 mmol/l) two hours after a 75 g oral glucose load as in a glucose tolerance test or symptoms of hyperglycemia and casual plasma glucose at or above 200 mg/dL (11.1 mmol/l).

As used herein, diabetes refers to "type 1 diabetes" also known as childhood-onset diabetes, juvenile diabetes, and insulin-dependent diabetes (IDDM) or "type 2 diabetes" also known as adult-onset diabetes, obesity-related diabetes, and non-insulin-dependent diabetes (NIDDM) or others forms of diabetes include gestational diabetes, insulin-resistant type 1 diabetes (or "double diabetes"), latent autoimmune diabetes of adults (or LADA) and maturity onset diabetes of the young (MODY) which is a group of several single gene (monogenic) disorders with strong family histories that present as type 2 diabetes before 30 years of age.

As used herein, a "diabetic disease state" refers to a pre-diabetic disease state, intermediate diabetic disease states characterized by stages of the disease more advanced then the pre-diabetic disease state and to disease states characteristic of overt diabetes as defined herein, including type I or II diabetes.

As used herein, a "pre-diabetic disease state" is one where a patient has an impaired fasting glucose level and impaired glucose tolerance. An impaired fasting glucose is defined as a blood glucose level from 100 to 125 mg/dL (6.1 and 7.0 mmol/l) i.e. an impaired fasting glucose. Patients with plasma glucose at or above 140 mg/dL or 7.8 mmol/l, but not over 200, two hours after a 75 g oral glucose load are considered to have impaired glucose tolerance.

As used herein, a "medical professional" is a physician or trained medical technician or nurse at a point-of-care facility.

A "point-of-care" facility can be at an inpatient location such as in a hospital or an outpatient location such as a doctor's office or a walk-in clinic. In one embodiment, the diagnostic assay may be distributed as a commercial kit to consumers together with instruments for the analysis of gene signature expression profile in a blood sample. In another embodiment, the commercial kit may be combined with instruments and reagents for the monitoring of blood glucose levels.

The term "blood glucose level" refers to the concentration of glucose in blood. The normal blood glucose level (euglycemia) is approximately 120 mg/dl. This value fluctuates by as much as 30 mg/dl in non-diabetics.

The condition of "hyperglycemia" (high blood sugar) is a condition in which the blood glucose level is too high. Typically, hyperglycemia occurs when the blood glucose level rises above 180 mg/dl.

As used herein, a "test sample" is any biological sample from a patient that contains cells that differentially express genes in response to a diabetic disease state. The biological sample can be any biological material isolated from an atopic or non-atopic mammal, such as a human, including a cellular component of blood, bone marrow, plasma, serum, lymph, cerebrospinal fluid or other secretions such as tears, saliva, or milk; tissue or organ biopsy samples; or cultured cells. Preferably the biological sample is a cellular sample that can be collected from a patient with minimal intervention. In a preferred embodiment, a test sample is a blood sample or a preparation of PBMCs (peripheral blood mononuclear cells) or CD14+ monocytes or CD11b+ or CD11c+ or Emr⁺ cells.

The mammal may be a human, or may be a domestic, companion or zoo animal. While it is particularly contemplated the herein described diagnostic tools are suitable for use in medical treatment of humans, they are also applicable to veterinary treatment, including treatment of companion animals such as dogs and cats, and domestic animals such as horses, cattle and sheep, or zoo animals such as non-human primates, felids, canids, bovids, and ungulates.

As used herein, the term "gene expression" refers to the process of converting genetic information encoded in a gene into RNA (e.g., mRNA, rRNA, tRNA, or snRNA) through "transcription" of the gene (e.g., via the enzymatic action of an RNA polymerise), and for protein encoding genes, into protein through "translation" of mRNA.

"Gene expression profile" refers to identified expression levels of at least one polynucleotide or protein expressed in a biological sample.

The term "primer" as used herein refers to an oligonucleotide either naturally occurring (e.g. as a restriction fragment) or produced synthetically, which is capable of acting as a point of initiation of synthesis of a primer extension product which is complementary to a nucleic acid strand (template or target sequence) when placed under suitable conditions (e.g. buffer, salt, temperature and pH) in the presence of nucleotides and an agent for nucleic acid polymerization, such as DNA dependent or RNA dependent polymerase. A primer must be sufficiently long to prime the synthesis of extension products in the presence of an agent for polymerization. A typical primer contains at least about 10 nucleotides in length of a sequence substantially complementary or homologous to the target sequence, but somewhat longer primers are preferred. Usually primers contain about 15-26 nucleotides.

As used herein, a "gene signature" refers to a pattern of gene expression of a selected set of genes that provides a unique identifier of a biological sample. A gene signature is diagnostic of a diabetic disease state if the pattern of gene expression of the selected set of genes is a substantial match to a gene signature in a reference sample taken from a patient with a diabetic disease state. For purposes of this application, a "gene signature" may be a pre-determined combination of nucleic acid or polypeptide sequences (if the genes are protein-coding genes). Gene signatures may comprise genes of unknown function or genes with no open reading frames including, but not limited to, rRNA, UsnRNA, microRNA or tRNAs.

As used herein, a "diagnostic gene expression profile" refers to the gene expression profile of a gene signature in a biological sample taken from a patient diagnosed with a particular disease state. The disease state can be a diabetic disease state or a non-diabetic disease state. A "substantial match" between a test gene expression profile from the patient and a diagnostic gene expression profile characteristic of a diabetic disease state indicates the patient has a diabetic disease state. Alternatively, a "substantial match" between a test gene expression profile from the patient and a diagnostic gene expression profile characteristic of a non-diabetic disease state indicates the patient does not have a diabetic disease state.

As used herein, a "variant" of a gene means gene sequences that are at least about 75% identical thereto, more preferably at least about 85% identical, and most preferably at least 90% identical and still more preferably at least about 95-99% identified when these DNA sequences are compared to a nucleic acid sequence of the prevalent wild type gene. In one embodiment, a variant of a gene is a gene with one or more alterations in the DNA sequence of the gene including, but not limited to, point mutations or single nucleotide polymorphisms, deletions, insertions, rearrangements, splice donor or acceptor site mutations and gene alterations characteristic of a pseudogenes. Throughout this application a gene implicitly includes both wild type and variants forms of the gene as defined herein.

As used herein, a "substantial match" refers to the comparison of the gene expression profile of a gene signature in a test sample with the gene expression profile of the gene signature in a reference sample taken from a patient with a defined disease state. The expression profiles are "substantially matched" if the expression of the gene signature in the test sample and the reference sample are at substantially the same levels. i.e., there is no statistically significant difference between the samples after normalization of the samples. In one embodiment, the confidence interval of substantially matched expression profiles is at least about 50% or from about 50% to about 75% or from about 75% to about 80% or from about 80% to about 85% or from about 85% to about 90% or from about 90% to about 95%. In a preferred embodiment, the confidence interval of substantially matched expression profiles is about 95% to about 100%. In another preferred embodiment, the confidence interval of substantially matched expression profiles is any number between about 95% to about 100%. In another preferred embodiment, the confidence interval of substantially matched expression profiles is about 95% or about 96% or about 97% or about 98% or about 99%, or about 99.9%.

As used herein, a "substantial difference" refers to the difference in the gene expression profile of a gene at one time point with the gene expression profile of the same gene signature at a second time point. The expression profiles are "substantially different" if the expression of the gene signature at the first and second time points are at different levels i.e. there is a statistically significant difference between the samples after normalization of the samples. In one embodiment, expression profiles are "substantially different" if the expression of the gene signature at the first and second time points are outside the calculated confidence interval. In one embodiment, the confidence interval of substantially different expression profiles is less than about 50% or less than about 75% or less than about 80% or less than from about 85% or less than about 90% or less than about 95%.

A 95% confidence interval CI is equal to AUC + 1.96 x standard error of AUC, where AUC is the area under the ROC Curve.

As used herein, "ROC" refers to a receiver operating characteristic, or simply ROC curve, which is a graphical plot of the sensitivity vs. (1 - specificity) for a binary classifier system as its discrimination threshold is varied.

As used herein, the terms "diagnosis" or "diagnosing" refers to the method of distinguishing one diabetic disease state from another diabetic disease state, or determining whether a diabetic disease state is present in an patient (atopic) relative to the "normal" or "non-diabetic" (non-atopic) state, and/or determining the nature of a diabetic disease state.

As used herein, "determining a diabetic disease state" refers to an integration of all information that is useful in diagnosing a patient with a diabetic disease state or condition and/or in classifying the disease. This information includes, but is not limited to family history, human genetics data, BMI, physical activity, metabolic disease profile and the results of a statistical analysis of the expression profiles of one or more gene signatures in a test sample taken from a patient. In the point-of-care setting, this information is analyzed and displayed by a computer system having appropriate data analysis software. Integration of the clinical data provides the attending physician with the information needed to determine if the patient has a diabetic condition, information related to the nature or classification of diabetes as well as information related to the prognosis and/or information useful in selecting an appropriate treatment. In one embodiment, the diagnostic assays, described herein, provide the medical professional with a determination of the efficacy of the prescribed medical treatment.

As used herein, a "metabolic disease profile" refers to any number of standard metabolic measures and other risk factors that can be diagnostic of a diabetic disease state including, but not limited to fasting plasma glucose, insulin, pro-insulin, c-peptide, intact insulin, BMI, waist circumference, GLP-1, adiponectin, PAI-1, hemoglobin A1c, HDL, LDL, VLDL, triglycerides, free fatty acids. The metabolic disease profile can be used to generate a superior model for classification equivalence to 2-hr OGTT.

A glucose tolerance test is the administration of glucose to determine how quickly it is cleared from the blood. The test is usually used to test for diabetes, insulin resistance, and sometimes reactive hypoglycemia. The glucose is most often given orally so the common test is technically an oral glucose tolerance test (OGTT).

The fasting plasma glucose test (FPG) is a carbohydrate metabolism test which measures plasma, or blood, glucose levels after a fast. Fasting stimulates the release of the hormone glucagon, which in turn raises plasma glucose levels. In people without diabetes, the body will produce and process insulin to counteract the rise in glucose levels. In people with diabetes this does not happen, and the tested glucose levels will remain high.

As used herein, the body mass index (BMI), or Quetelet index, is a statistical measurement which compares a person's weight and height. Due to its ease of measurement and calculation, it is the most widely used diagnostic tool to identify obesity.

As used herein, NGT means Normal Glucose Tolerance, IGT means Impaired Glucose Tolerance and T2D means type 2 diabetes.

As used herein, CD11c⁺, CD11b⁺ and Emr⁺ are cell surface markers of human monocyte/macrophage and myeloid cells and their precursors. In mice, the most commonly used monocyte/macrophage and myeloid cell surface markers are F4/80 and CD11b, although F4/80 and CD11b antibodies have been reported to react with eosinophils and dendritic cells and NK and other T and B cell subtypes, respectively (Nguyen, et al. (2007) J Biol Chem 282, 35279-35292; Patsouris, et al. (2008) Cell Metab. 8, 301-309). The F4/80 gene in mouse is the ortholog to the human Emr1 gene. The human ortholog for the mouse CD11c gene is ITGAX also called integrin, alpha X (complement component 3 receptor 4 subunit), SLEB6, OTTHUMP00000163299; leu M5, alpha subunit; leukocyte surface antigen p150,95, alpha subunit; myeloid membrane antigen, alpha subunit; p150 95 integrin alpha chain (Chromosome: 16; Location: 16p11.2 Annotation: Chromosome 16, NC_000016.8 (31274010..31301819) MIM: 151510, GeneID:3687). The human ortholog for the mouse CD11b gene is ITGAM or integrin, alpha M (complement component 3 receptor 3 subunit) also called CD11B, CR3A, MAC-1, MAC1A, MGC117044, MO1A, SLEB6, macrophage antigen alpha polypeptide; neutrophil adherence receptor alpha-M subunit (Chromosome: 16; Location: 16p11.2 Chromosome 16, CNC_000016.8 (31178789..31251714), MIM: 120980, GeneID: 3684). CD11c⁺, CD11b⁺ and Emr⁺ and CD14⁺ cells can be purified from PBMCs by positive selection using the appropriate human blood cell isolation kit (StemCell Technologies). Purity of isolated cells populations (>85%) is then confirmed by flow cytometry staining of fluorescent-conjugated antibodies to the appropriate cell surface mnrker (BioLegend).

As used herein, "real-time PCR" refers to real-time polymerase chain reaction, also called quantitative real time polymerase chain reaction (Q-PCR/qPCR) or kinetic polymerase chain reaction. Real-time PCR is a laboratory technique based on the polymerase chain reaction, which is used to amplify and simultaneously quantify a targeted DNA molecule. It enables both detection and quantification (as absolute number of copies or relative amount when normalized to DNA input or additional normalizing genes) of a specific sequence in a DNA sample.

As used herein, an immunochemical assay is a biochemical test that measures the concentration of a substance in a cellular extract using the reaction of an antibody or antibodies to its antigen. In this disclosure, the antigen is a protein expressed by anyone of the protein coding genes comprising a gene signature. In a preferred embodiment, the immunochemical assay is an Enzyme-Linked ImmunoSorbent Assay (ELISA).

As used herein, "specific oligonucleotide hybridization" refers to hybridization between probe sequences on a solid support such as a chip and cDNA sequences generated from transcripts within the patient's test sample. If the two nucleic acid sequences are substantially complementary, hybridization occurs which is directly proportional to the amount of cDNA sequences in the test sample. Detection of hybridization is then achieved using techniques well known in the art. Numerous factors influence the efficiency and selectivity of hybridization of two nucleic acids, for example, a nucleic acid member on a array, to a target nucleic acid sequence. These factors include nucleic acid member length, nucleotide sequence and/or composition, hybridization temperature, buffer composition and potential for steric hindrance in the region to which the nucleic acid member is required to hybridize. A positive correlation exists between the nucleic acid member length and both the efficiency and accuracy with which a nucleic acid member will anneal to a target sequence. In particular, longer sequences have a higher melting temperature (TM) than do shorter ones, and are less likely to be repeated within a given target sequence, thereby minimizing promiscuous hybridization. Hybridization temperature varies inversely with nucleic acid member annealing efficiency, as does the concentration of organic solvents, e.g., formamide, that might be included in a hybridization mixture, while increases in salt concentration facilitate binding. Under stringent annealing conditions, longer nucleic acids, hybridize more efficiently than do shorter ones, which are sufficient under more permissive conditions.

As used herein, the term "antibody" includes both polyclonal and monoclonal antibodies; and may be an intact molecule, a fragment thereof (such as Fv, Fd, Fab, Fab' and F(ab)'2 fragments, or multimers or aggregates of intact molecules and/or fragments; and may occur in nature or be produced, e.g., by immunization, synthesis or genetic engineering.

As used herein, all references to probes in Tables 1, 7, 8, 9, 10A and 10B refer to probe sets represented on the GeneChip Human Genome U133 Plus 2.0 Array.

The following description relates to certain embodiments of the application, and to a particular methodology for diagnosing a diabetic disease state in a patient. In particular, the application discloses a number of genes, including some which had not previously been considered to be associated with a diabetic disease state, are differentially expressed in peripheral blood mononuclear cells (PMBC) from patients which have a diabetic or pre-diabetic disease state as compared to patients who do not have a diabetic disease state.

In one embodiment, genes that are differentially expressed in PBMCs of NGTs and T2Ds are identified using microarray analysis. Transcripts from PBMCs of NGT and T2D patients (in this example, a cohort of 107 patients) were initially screened using the Affymetrix Human Genome HG-U133Plus2 chip, according to the manufacturer's instructions. Approximately 200 differentially expressed genes were selected which had a False Discovery Rate, FDR<20%, fold change >1.7 between NGTs and T2Ds using the Significance Analysis of Microarray (SAM) program (see TABLE 1).

Methods of diabetes classification are now described by determining the differential expression of different combinations of diabetes susceptibility genes identified in the initial microarray screen (see TABLE 12A). Table 12A also includes the Genbank Accession Numbers of each of the selected genes.

Gene expression of diabetes susceptibility genes may be measured in a biological sample using a number of different techniques. For example, identification of mRNA from the diabetes-associated genes within a mixture of various mRNAs is conveniently accomplished by the use of reverse transcriptase-polymerase chain reaction (RT-PCR) and an oligonucleotide hybridization probe that is labeled with a detectable moiety.

First a test sample is collected from a patient. To obtain high quality RNA it is necessary to minimize the activity of RNase liberated during cell lysis. This is normally accomplished by using isolation methods that disrupt tissues and inactivate or inhibit RNases simultaneously. For specimens low in endogenous ribonuclease, isolation protocols commonly use extraction buffers contain detergents to solubilize membranes, and inhibitors of RNase such as placental ribonuclease inhibitor or vanadylribonucleoside complexes. RNA isolation from more challenging samples, such as intact tissues or cells high in endogenous ribonuclease, requires a more aggressive approach. In these cases, the tissue or cells are quickly homogenized in a powerful protein denaturant (usually guanidinium isothiocyanate), to irreversibly inactivate nucleases and solubilize cell membranes. If a tissue sample can not be promptly homogenized, it must be rapidly frozen by immersion in liquid nitrogen, and stored at -80° C. Samples frozen in this manner must never be thawed prior to RNA isolation or the RNA will be rapidly degraded by RNase liberated during the cell lysis that occurs during freezing. The tissue must be immersed in a pool of liquid nitrogen and ground to a fine powder using mortar and pestle. Once powdered, the still-frozen tissue is homogenized in RNA extraction buffer. A number of kits for RNA isolation are now commercially available (Ambion, Quiagen).

As is well known in the art, cDNA is first generated by first reverse transcribing a first strand of cDNA from a template mRNA using a RNA dependent DNA polymerase and a primer. Reverse transcriptases useful according to the application include, but are not limited to, reverse transcriptases from HIV, HTLV-1, HTLV-II, FeLV, FIV, SIV, AMV, MMTV, MoMuLV and other retroviruses (for reviews, see for example, Levin, 1997, Cell 88:5-8; Verma, 1977, Biochim. Biophys. Acta 473:1-38; Wu et al., 1975, CRC Crit. Rev. Biochem. 3:289-347). More recently, a number of kits are now commercially available for RT-PCR reactions using thermostable reverse transcriptase, e.g. GeneAmp® Thermostable rTth Reverse Transcriptase RNA PCR Kit (Applied Biosystems).

"Polymerase chain reaction," or "PCR," as used herein generally refers to a method for amplification of a desired nucleotide sequence in vitro, as described in U.S. Patent Nos. 4,683,202, 4,683,195, 4,800,159, and 4,965,188. The PCR reaction involves a repetitive series of temperature cycles and is typically performed in a volume of 10-100 µl. The reaction mix comprises dNTPs (each of the four deoxynucleotides dATP, dCTP, dGTP, and dTTP), primers, buffers, DNA polymerase, and nucleic acid template. The PCR reaction comprises providing a set of polynucleotide primers wherein a first primer contains a sequence complementary to a region in one strand of the nucleic acid template sequence and primes the synthesis of a complementary DNA strand, and a second primer contains a sequence complementary to a region in a second strand of the target nucleic acid sequence and primes the synthesis of a complementary DNA strand, and amplifying the nucleic acid template sequence employing a nucleic acid polymerase as a template-dependent polymerizing agent under conditions which are permissive for PCR cycling steps of (i) annealing of primers required for amplification to a target nucleic acid sequence contained within the template sequence, (ii) extending the primers wherein the nucleic acid polymerase synthesizes a primer extension product.

Other methods of amplification include, but are not limited to, ligase chain reaction (LCR), polynucleotide-specific based amplification (NSBA).

Primers can readily be designed and synthesized by one of skill in the art for the nucleic acid region of interest. It will be appreciated that suitable primers to be used with the application can be designed using any suitable method. Primer selection for PCR is described, e.g., in U.S. Pat. No. 6,898,531, issued May 24, 2005, entitled "Algorithms for Selection of Primer Pairs" and U.S. Ser. No. 10/236,480, filed Sep. 5, 2002; for short-range PCR, U.S. Ser. No. 10/341,832, filed Jan. 14, 2003 provides guidance with respect to primer selection. Also, there are publicly available programs such as "Oligo", LASERGENE®, primer premier 5 (available at the website of the company Premier Biosoft) and primer3 (available at the website of the Whitehead Institute for Biomedical Research, Cambridge, Mass., U.S.A). Primer design is based on a number of parameters, such as optimum melting temperature (Tm) for the hybridization conditions to be used and the desired length of the oligonucleotide probe. In addition, oligonucleotide design attempts to minimize the potential secondary structures a molecule might contain, such as hairpin structures and dimmers between probes, with the goal being to maximize availability of the resulting probe for hybridization. In a preferred embodiment, the primers used in the PCR method will be complementary to nucleotide sequences within the cDNA template and preferably over exon-intron boundaries.

In one embodiment, the PCR reaction can use nested PCR primers.

In one embodiment, a detectable label may be included in an amplification reaction. Suitable labels include fluorochromes, e.g. fluorescein isothiocyanate (FITC), rhodamine, Texas Red, phycoerythrin, allophycocyanin, 6-carboxyfluorexcein (6-FAM), 2',7'-dimethoxy-4',5'-dichloro-5-carboxyfluorescein (JOE), 6-carboxy-X-rhodamine(ROX), 6-carboxy-2,4',7',4,7-hexachloeofluorescein (HEX), 5-carboxyfluorescein (5-FAM) or N,N,N',N'-tetramethyl-6-carboxyrhodamine (TAMRA), radioactive labels, e.g. 32P, 35S, 3H; as well as others. The label may be a two stage system, where the amplified DNA is conjugated to biotin, haptens, or the like having a high affinity binding partner, e.g. avidin, specific antibodies, etc., where the binding partner is conjugated to a detectable label. The label may be conjugated to one or both of the primers. Alternatively, the pool of nucleotides used in the amplification is labeled, so as to incorporate the label into the amplification product.

In a particularly preferred embodiment the application utilizes a combined PCR and hybridization probing system so as to take advantage of assay systems such as the use of FRET probes as disclosed in US patents U.S. Pat. Nos. 6,140,054 and 6,174,670. In one of its simplest configurations, the FRET or "fluorescent resonance energy transfer" approach employs two oligonucleotides which bind to adjacent sites on the same strand of the nucleic acid being amplified. One oligonucleotide is labeled with a donor fluorophore which absorbs light at a first wavelength and emits light in response, and the second is labeled with an acceptor fluorophore which is capable of fluorescence in response to the emitted light of the first donor (but not substantially by the light source exciting the first donor, and whose emission can be distinguished from that of the first fluorophore). In this configuration, the second or acceptor fluorophore shows a substantial increase in fluorescence when it is in close proximity to the first or donor fluorophore, such as occurs when the two oligonucleotides come in close proximity when they hybridise to adjacent sites on the nucleic acid being amplified, for example in the annealing phase of PCR, forming a fluorogenic complex. As more of the nucleic acid being amplified accumulates, so more of the fluorogenic complex can be formed and there is an increase in the fluorescence from the acceptor probe, and this can be measured. Hence the method allows detection of the amount of product as it is being formed.

It will also be appreciated by those skilled in the art that detection of amplification can be carried out using numerous means in the art, for example using TaqMan™ hybridisation probes in the PCR reaction and measurement of fluorescence specific for the target nucleic acids once sufficient amplification has taken place. TaqMan Real-time PCR measures accumulation of a product via the fluorophore during the exponential stages of the PCR, rather than at the end point as in conventional PCR. The exponential increase of the product is used to determine the threshold cycle, CT, i.e. the number of PCR cycles at which a significant exponential increase in fluorescence is detected, and which is directly correlated with the number of copies of DNA template present in the reaction.

Although those skilled in the art will be aware that other similar quantitative "real-time" and homogenous nucleic acid amplification/detection systems exist such as those based on the TaqMan approach (see U.S. Pat. Nos. 5,538,848 and 5,691,146),
fluorescence polarisation assays (e.g. Gibson et al., 1997, Clin Chem., 43: 1336-1341), and the Invader assay (e.g. Agarwal et al., Diagn Mol Pathol 2000 September; 9(3): 158-164; Ryan D et al, Mol Diagn 1999 June; 4(2): 135-144). Such systems would also be adaptable for use in the described application, enabling real-time monitoring of nucleic acid amplification.

In another embodiment of the application, matrices or microchips are manufactured to contain an array of loci each containing a oligonucleotide of known sequence. In this disclosure, each locus contains a molar excess of selected immobilized synthetic oligomers synthesized so as to contain complementary sequences for desired portions of a diabetes susceptibility gene. Transcripts of diabetes susceptibility genes present in PBMC are amplified by RT-PCR and labeled, as described herein. The oligomers on the microchips are then hybridized with the labeled RT-PCR amplified diabetes susceptibility gene nucleic acids. Hybridization occurs under stringent conditions to ensure that only perfect or near perfect matches between the sequence embedded in the microchip and the target sequence will occur during hybridization. The resulting fluorescence at each locus is proportional to the expression level of the one or more diabetes susceptibility gene in the PBMCs.

In other embodiments of the application, gene signature expression profiles of protein-coding genes are determined using techniques well known in the art of immunochemistry including, for example, antibody-based binding assays such as ELISA or radioimmunoassays or protein arrays containing antibodies directed to the protein products of genes within a pre-determined signature as defined herein.

In one embodiment, the expression profiles of the TCF7L2 and CLC genes were analyzed in peripheral blood mononuclear cells from normal glucose tolerant and type 2 diabetic patients.

The human Charcot-Leyden crystal protein gene is expressed primarily in eosinophils. CLC is down regulated sequentially in PBMC of NGTs to IGTs to T2Ds. The mean signal intensities of its expression in microarray of the 107-patient cohort are listed In TABLE 2 below. Receiver operating characteristic (ROC) analysis demonstrated that the CLC gene-expression level can be used to separate NGTs from IGTs/T2Ds.

**TABLE 2: CLC gene expression in PBMCs isolated from NGTs, IGTs and T2Ds**

| **NGT** | **IGT** | **T2D** |
|---|---|---|
| 1504 | 900 | 410 |

The performance of CLC gene in predicting the clinical status was further examined using a receiver operating characteristic (ROC) analysis. An ROC curve shows the relationship between sensitivity and specificity. That is, an increase in sensitivity will be accompanied by a decrease in specificity. The closer the curve follows the left axis and then the top edge of the ROC space, the more accurate the test. Conversely, the closer the curve comes to the 45-degree diagonal of the ROC graph, the less accurate the test. The area under the ROC is a measure of test accuracy. The accuracy of the test depends on how well the test separates the group being tested into those with and without the disease in question. An area under the curve (referred to as "AUC") of 1 represents a perfect test, while an area of 0.5 represents a less useful test. Thus, preferred genes and diagnostic methods of the present application have an AUC greater than 0.50, more preferred tests have an AUC greater than 0.60, more preferred tests have an AUC greater than 0.70.

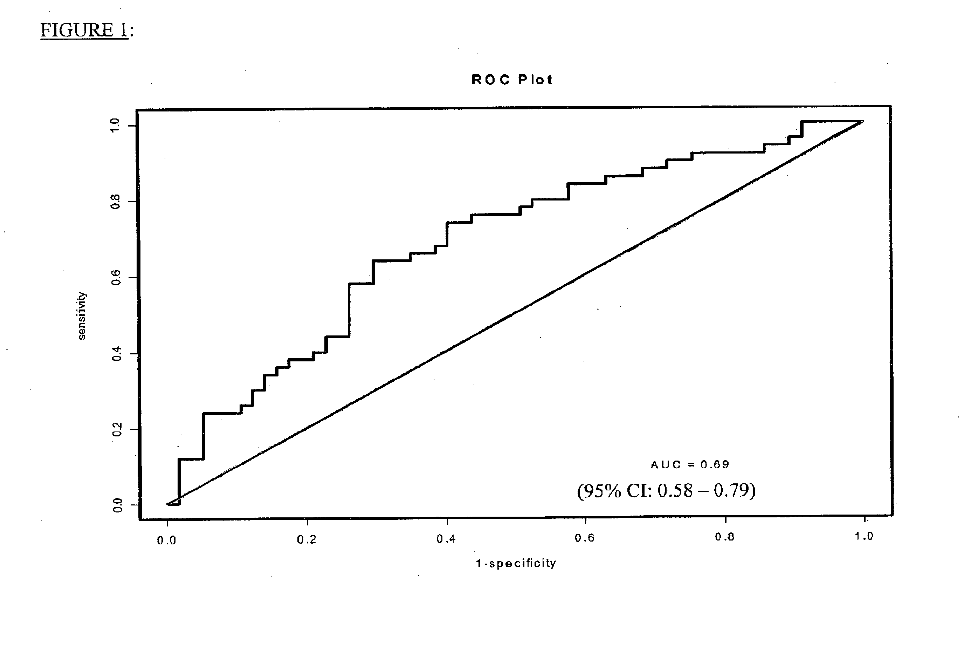

The area under curve (AUC) was calculated as a measure of the performance of CLC gene in predicting patient status. Receiver operating characteristic (ROC) analysis of the CLC gene date demonstrated that the CLC gene expression level can be used to separate NGTs from IGTs/T2Ds (see FIG. 1).

Genetic variants in the gene encoding for transcription factor-7-like 2 (TCF7L2) have been strongly associated with a risk for developing type 2 diabetes and impaired, β-cell insulin function (see US 2006/0286588 ). Genome-wide association studies implicate SNPs within TCF7L2 give the highest lifetime risk score for predicting type 2 diabetes progression compared to SNPs in other marker genes, including CDKAL1, CDKN2A/2B, FTO, IGF2BP2, and SLC30A8 (range of risk scores 1.12 - 1.20). TCF7L2 is widely expressed and this transcription factor is known to respond to developmental signals from members of the Wnt family of proteins. Functional and genetic studies point to a critical role for TCF7L2 in the development of the intestine and proglucagon gene expression in enteroendocrine cells.

To ascertain if TCF7L2 and the CLC gene are diagnostic markers of diabetes, either individually or in combination, 180 subjects were recruited from the German population in association with the Institute for Clinical Research & Development (IKFE), in Mainz, Germany. Appropriate IRB approvals were obtained prior to patient sample collection. The inclusion criteria consisted of patients between 18-75 years and a body-mass index (BMI) ≥ 30 who had no previous diagnosis of diabetes, and the legal capacity and ability to understand the nature and extent of the clinical study and the required procedures. The exclusion criteria consisted of blood donation within the last 30 days, insulin dependent diabetes mellitus, lactating or pregnant women, or women who intend to become pregnant during the course of the study, sexually active women not practicing birth control, history of severe/ multiple allergies, drug or alcohol abuse, and lack of compliance to study requirements. All clinical measurements including the 75g-oral glucose tolerance test results (OGTT) were obtained using standard procedures.

Blood samples were drawn by venipuncture into CPT tubes (BD Biosciences). PBMCs were isolated according to manufacturer's protocol and the final cell pellet was resuspended in 1ml of Trizol (Invitrogen), and stored at -80°C. Subsequently, total RNA was purified using manufacturer's protocol and resuspended in DEPC-treated ddH₂O. RNA quantification and quality was performed using the ND-1000 Spectrophotometer (NanoDrop) and reconfirmed by spectrophotometric quantitation with RiboGreen kit (Molecular Probes). The quality of RNA templates was measured by using the Bioanalyzer 2100 (Agilent Technologies).

First-strand cDNA synthesis was performed using 200 ng of total RNA from each patient PBMC sample using the High Capacity cDNA Reverse Transcription kit

(Applied Biosystems). Afterwards, the reaction mixture was diluted 10-fold with ddH₂O. and 4 µl was used as template in a 10 µl Taqman PCR reaction on the ABI Prism 7900HT sequence detection system. The reaction components consisted of 2X Taqman PCR master mix (Applied Biosystems), 0.9 µM of each primer, and 0.25 µM of fluorescent-labeled probe (Biosearch Technologies). Sequences for primer/probe sets used in RT-PCR Taqman assay are presented in **Table 3.**

### Cycling conditions for reverse transcription step were as follows:

| | **Step 1** | **Step 2** | **Step 3** | **Step 4** |
|---|---|---|---|---|
| **Temp (C°)** | 25 | 37 | 85 | 4 |
| **Time** | 10 min. | 120 min. | 5 sec. | ∞ |

**Table 3. Probe and Primer sequences used in Taqman assays for TCF7L2, CLC and ACTIN**

| **Marker set #** | **5'>3' -Sequence** | **Sequence Name** | **Comment** |
|---|---|---|---|
| TCF7L2 set 1 | ACCTGAGCGCTCCTAAGAAATG | TCF7L2_DL_F1 | NOT over junction |
| | AGGGCCGCACCAGTTATTC | TCF7L2_DL_R1 | NOT over junction |
| | FAM-AGCGCGCTTTGGCCTTGATCAAC-BHQ1 | TCF7L2_DL_Pro1 | NOT over junction |
| | | | |
| TCF7L2 set 2 | CGTCGACTTCTTGGTTACATTCC | TCF7L2_DL_F2 | NOT over junction |
| | CACGACGCTAAAGCTATTCTAAAGAC | ICF7L2_DL_R2 | NOT over junction |
| | FAM- CAGCCGCTGTCGCTCGTCACC-BHQ1 | ICF7L2_DL_Pro2 | NOT over junction |
| | | | |
| TCF7L2 set 3 | GAAAGCGCGGCCATCAAC | TCF7L2_1564_U18 | Over junction |
| | GAGCTCGTAGTATTTCGCTTGCT | TCF7L2_1644_L23 | Over junction |
| | FAM-TCCTTGGGCGGAGGTGGCATG-BHQ1 | TCF7L2_1586_P21 | Over junction |
| | | | |
| CLC set 1 | GCTACCCGTGCCATACACAGA | CLC_85_U21 | Over junction |
| | GCAGATATGGTTCATTCAAGAAACA | CLC_185_L25 | Over junction |
| | FAM-TTCTACTGTGACAATCAAAGGGCGACCA-BHQ1 | CLC_127_P28 | Over junction |
| | | | |
| **ACTIN** | CCTGGCACCCAGCACAAT | B-actin-1F | Internal Control |
| | GCCGATCCACACGGAGTACTT | B-actin-1R | |
| | FAM-ATCAAGATCATTGCTCCTCCTGAGCGC-BHQ1 | B-actin P | |

Quantitative real-time RT-PCR by Taqman assay using two different primer/ probe combinations specific for TCF7L2 and one prime/probe set for CLC, was performed on RNA isolated from PBMCs from individual patients. Thermocycling profile for PCR step was as follows, 95°C for 10 min, followed by 40 cycles of 95°C (15 sec) and 60°C (1 min). Ct values were calculated from the raw data using the software SDS version 2.1 (Applied Biosystems), with threshold set at 0.2 to 0.3. Run-to run reproducibility by Pearson correlation was R²=0.96-0.98 for the above-mentioned markers. The delta Ct (cycle threshold) value was calculated by subtracting the Ct of the housekeeping β-actin gene from the Ct of marker of interest, for instance, TCF7L2 (Ct TCF7L2 - Ct actin). The value of [2^{-(delta Ct)} x 1000] was used to represent the expression of TCF7L2 relative to β-action. The OGTT result was used as the true clinical status. Student's T-test was used for determining statistical significance between expression levels of gene markers using normalized Ct values.

Based on the 2-hr OGTT measurement and current ADA guidelines, of the 180 patients enrolled in the study, 104 patients were classified as NGT (Normal Glucose Tolerance), 49 patients as IGT (impaired glucose tolerance) and 27 patients were considered T2D (type 2 diabetes). Because the T2D subjects were diagnosed with diabetes for the first time in this study, the duration of the disease for each patient, and how long the PBMCs were sustained in a hyperglycemic microenvironment was unknown.

T-test analysis for expression levels of TCF7L2 and CLC normalized to β-actin for each patient, based on primer/probe sets values and separated by glucose tolerance, is depicted in Table 4. The NGT and IGT plus T2D patient groups had a statistically significant difference between expression levels by Student's T-test, with p-values =0.004; 0.021 and 0.022 for TCF7L2 set 1, set 3 and CLC, respectively. These results indicate differential expression of the TCF7L2 and CLC genes in PBMCs of pre-diabetic (IGT) patients or pre-diabetic and T2D patients combined together compared to NGT.

**TABLE 4: Statistical difference in expression levels of TCF7L2 and CLC by Student's T-test**

| **Sample n** | **T-test** | **Bactin Ct** | **Normalized TCF7L2_1** | **Normalized TCF7L2_2** | **Normalized TCF7L2_3** | **Normalized CLC** |
|---|---|---|---|---|---|---|
| 104/49 | **NGT/IGT** | 0.451 | 0.005 | 0.075 | 0.008 | 0.013 |
| **104/76** | **NGT/(IGT+T2D)** | 0.277 | **0.004** | 0.093 | **0.021** | **0.022** |
| | **Statistically significant differentiation** | | | | | |

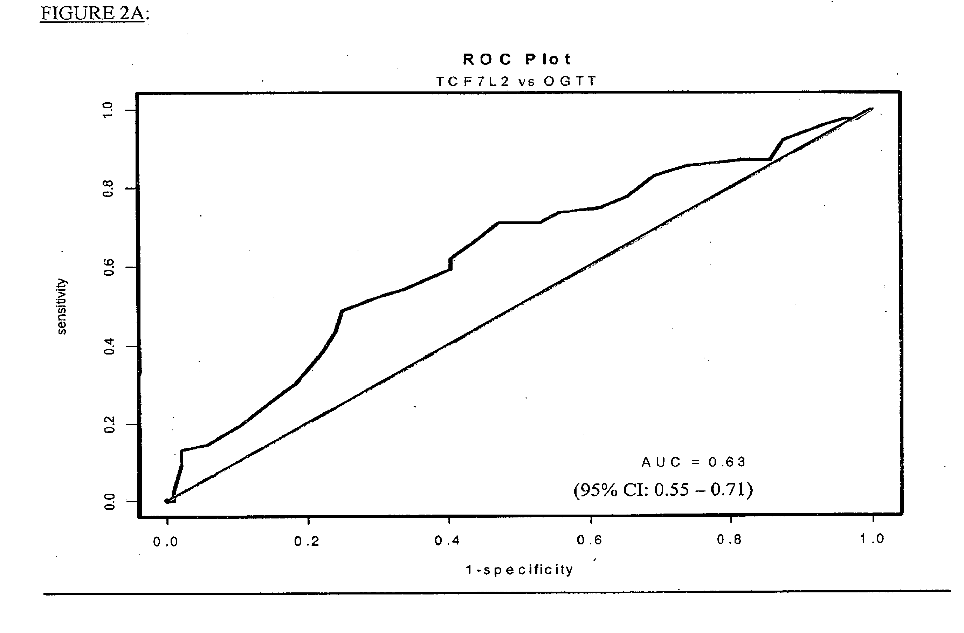

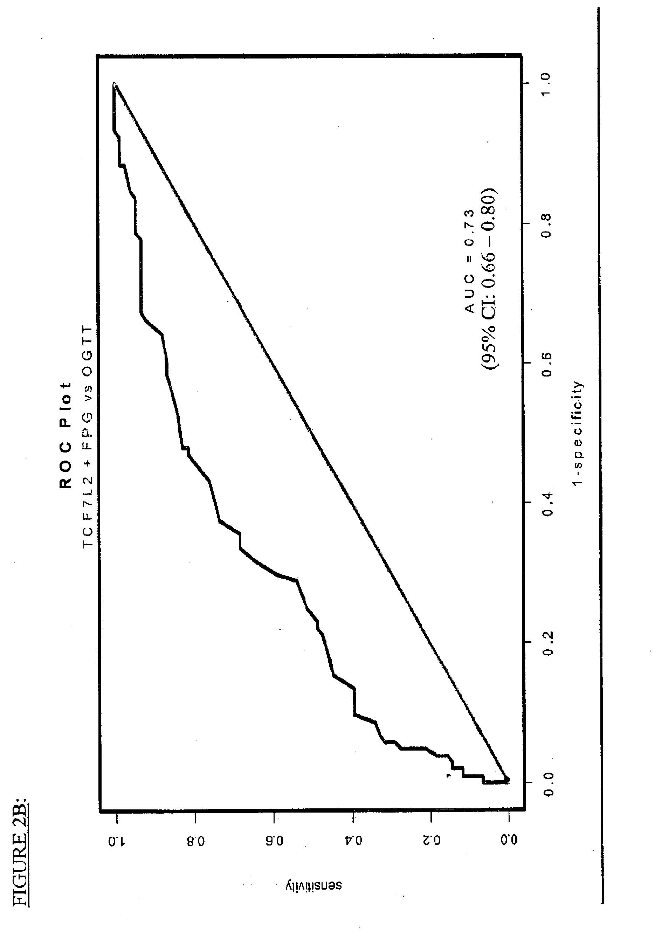

Next, the performance of the TCF7L2 and CLC Taqman assays as a diagnostic tool for the classification of patients as normal or pre-diabetic/diabetic, compared to the 2-hr OGTT was assessed. Receiver Operating Characteristic (ROC) curves for each TCF7L2 and CLC primer/probe set normalized delta Ct value were generated (Table 5, FIGs. 2A and 2B). The AUC values for the TCF7L2 set 1 and CLC PCR assays were 0.63 and 0.61, respectively. Compared to the 2-hr OGTT classification, TCF7L2 set 1 expression from PBMCs can correctly classify a patient as being normal or pre-diabetic/diabetic with an AUC of 0.73 when used in conjunction with the FPG test (FIGs. 2A and 2B). CLC did not have an additive value to TCF7L2 set 1 and was not considered for the diagnostic algorithm. Additionally, exclusion of 14 patients that had FPG ≥ 126mg/dL (also considered diabetic) did not change the performance of the assay.

**Table 5: ROC curve AUC values for each marker probe-primer set and in combination with FPG values**

| | **ROC AUC value** | |
|---|---|---|
| **Marker set #** | **Marker vs. OGTT** | **Marker+FPG vs. OGTT** |
| **TCF7L2 set 1** | 0.63 | **0.73** |
| **TCF7L2 set 2** | 0.59 | ND |
| **TCF7L2 set 3** | 0.61 | 0.72 |
| **CLC set 1** | 0.60 | 0.69 |

In one embodiment, each of the genes selected in the microarray analysis (see TABLE 1) may be combined with the performance of TCF7L2 set 1 to more closely match the 2-hr OGTT result.

In another embodiment, genes that are strongly associated with a risk of type 2 diabetes (see TABLE 6) may also be combined with the performance of TCF7L2 set 1 to more closely match the 2-hr OGTT result. In another embodiment, the genes of Table 6 in combination with one or more genes of TABLE 1 can be tested as described herein for gene signatures that are diagnostic of a diabetic disease state.

**TABLE 6:**

| Gene Symbols | | | |
|---|---|---|---|
| NOTCH2 | IGF2BP2 | LGR5 | CDKN2A-2B |
| THADA | WFS1 | FTO | HHEX-IDE |
| PPARG | KCNJ11 | JAZF1 | CDC123 |
| ADAMTS9 | TSPAN8 | SLC30A8 | CAMK1D |

In another embodiment, the expression of CDKN1C, a member of the CIP/KIP family was also differentially expressed in PBMCs from NGTs and T2Ds.

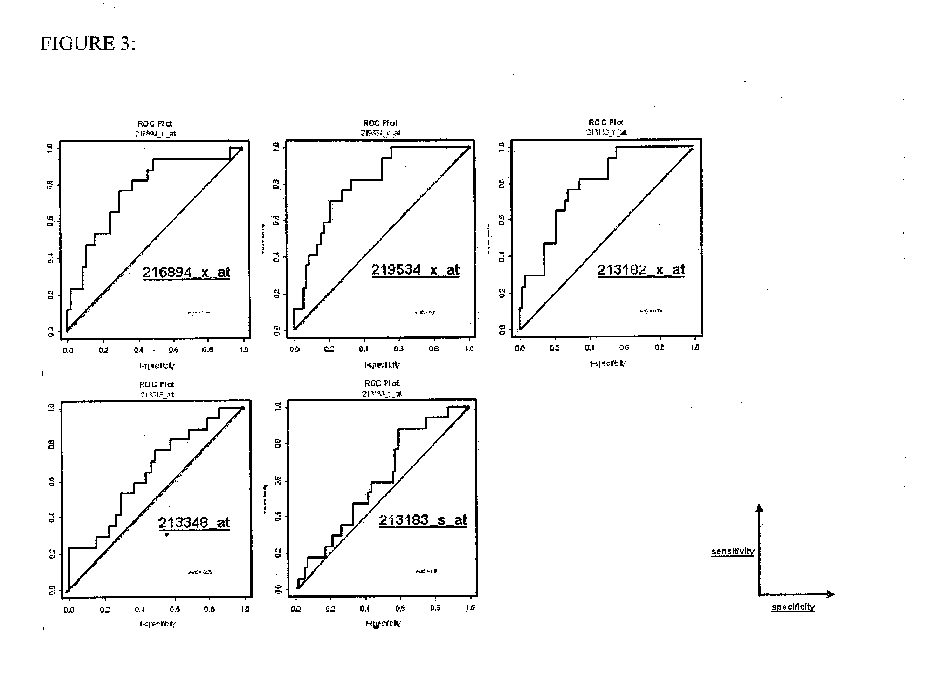

The CIP/KIP family consists of three members, CDKN2A, CDKN2B and CDKN1C. All of the three members can inhibit the activity of CDK4, which plays a central role in regulating mammalian cell cycle. Islet β-cell replication plays an essential role in maintaining β-cell mass homeostasis. It has been known that CDK4 has an important role in the regulation of body weight and pancreatic β-cell proliferation. In mice, loss of the CDK4 gene resulted in insulin-deficient diabetes due to the reduction of β-cell mass whereas activation of CDK4 caused β-islet cell hyperplasia. Recently, genome-wide association studies of type 2 diabetes have revealed that nucleotide variation near CDKN2A and CDKN2B genes is associated with type 2 diabetes risk. In addition, over-expression of CDKN2A leads to decreased islet proliferation in aging mice and over-expression of CDKN2B is related to islet hypoplasia and diabetes in murine models. CDKN1C is a maternally expressed gene located on chromosome 11p15.5 and is involved in the pathogenesis of Beckwith-Wiedemann syndrome (BWS), a disorder characterized by neonatal hyperinsulinemic hypoglycemia, as well as pre- and postnatal overgrowth. Recent studies also showed that CDKN1C is down-regulated by insulin and variants of CDKN1C may be associated to increased birth weights in type 2 diabetes patients. In addition to regulating the cell cycle, the CIP/KIP family plays an important role in other biological processes, such as apoptosis, transcription regulation, differentiation and cell migration. The expression of the three genes in the 107 patient cohort was analyzed. Only CDKN1C displayed differential expression among NGTs, IGTs and T2Ds (see TABLE 7). There are 5 probes expressing in PBMC for CDKN1C on the HG-U133Plus2 GeneChip. Each of them displayed differential expression between NGTs and IGTs/T2Ds (TABLE 7). ROC analysis showed that expression levels of the 5 probes can be used to separate NGTs from T2Ds (FIG. 3).

**TABLE 7:**

| **CDKN1C gene expression in PBMCs isolated from NGTs, IGTs and T2Ds** | | | | | |
|---|---|---|---|---|---|
| **Gene** | **Probe** | **Expression in PBMC** | **Mean**_**NGT** | **Mean_IGT** | **Mean_T2D** |
| CDKN1C | 213182_x_at | Yes | 935 | 1178 | 1784 |
| | 213183_s_at | Yes | 531 | 712 | 624 |
| | 213348_at | Yes | 2648 | 3246 | 3957 |
| | 216894_x_at | Yes | 797 | 1030 | 1439 |
| | 219534_x_at | Yes | 1092 | 1356 | 1973 |

In a person of ordinary skill in the art will recognize that the described embodiments provide a premise to investigate gene signatures as a diagnostic tool of diabetes. To investigate the underlying biological processes between normal subjects and pre-diabetes and diabetes patients, pathway analysis was conducted. Namely, the probes on HG-U133Plus2 chip were mapped to Gene Ontology Biological Process (GOBP) as described by Yu et al. BMC Cancer 7:182 (2007). Since genes with very low expression tend to have higher variations, genes whose mean intensity is less than 200 in the dataset were removed from pathway analysis. As a result, 21247 probes were retained. To identify pathways that have significant association with the development of pre-diabetes or diabetes, global test program was run by comparing NGT vs. IGT, NGT vs. T2D, or NGT vs. IGT+T2D. The pathways that have at least 10 probes and a significant p value (p < 0.05) were identified for each comparison. There were 3 pathways that had consistent association with the patient outcomes through the three comparisons. They are B cell activation (GO0042113), humoral immune response (GO0006959), and DNA unwinding during replication (GO0006268). Among the 3 pathways, B cell activation and humoral immune response have dominantly negative association with diabetes (lower expression in IGT/T2D) whereas DNA unwinding during replication has positive association with diabetes (higher expression in IGT/T2D).

To build a pathway-based gene signature from the 3 key pathways, genes with a p < 0.05 were pooled and sorted based on their statistical significance (z score from Global Test). If a gene has more than one probe in the list and their behaviors were consistent, the one with the highest significance was retained. If a gene has more than one probe in the list and their behaviors were opposite, all probes for this gene were removed. As a result, 14 unique genes were obtained (SEE TABLE 8 below).

**TABLE 8: Pathway Significant Genes**

| **PSID** | **Gene Symbol** | **Gene Title** |
|---|---|---|
| 208900_s_at | **TOP1** | topoisomerase (DNA) I |
| 216379_x_at | **CD24** | CD24 antigen (small cell lung carcinoma cluster 4 antigen) |
| 222430_s_at | **YTHDF2** | YTH domain family, member 2 |
| 1554343_a_at | **BRDG1** | BCR downstream signaling 1 |
| 228592_at | **MS4A1** | membrane-spanning 4-domains, subfamily A, member 1 |
| 216894_x_at | **CDKN1C** | cyclin-dependent kinase inhibitor 1C (p57, Kip2) |
| 1558662_s_at | **BANK1** | B-cell scaffold protein with ankyrin repeats 1 |
| 205267_at | **POU2AF1** | POU domain, Gass 2, associating factor 1 |
| 205859_at | **LY86** | lymphocyte antigen 86 |
| 221969_at | **PAX5** | Paired box gene 5 (B-cell lineage specific activator) |
| 207655_s_at | **BLNK** | B-cell linker |
| 206126_at | **BLR1** | Burkitt lymphoma receptor 1, GTP binding protein (chemokine (C-X-C motif) receptor 5) |
| 206983_at | **CCR6** | chemokine (C-C motif) receptor 6 |
| 204946_s_at | **TOP3A** | topoisomerase (DNA) III alpha |
| 214252_s_at | **CLN5** | ceroid-lipofuscinosis, neuronal 5 |

To build a signature using genes with relatively high variation, 10 genes with a CV > 0.25 were retained. To determine the optimal number of genes for a signature, combination of top 2 - 10 genes were examined in the dataset. The result indicated that the top 3 genes gave the best performance in the prediction of patients' outcomes. The 3 genes, TOP1, CD24 and STAP1 below in TABLE 9.

**TABLE 9:**

| **3 gene expression in PBMCs isolated from NGTs, IGTs and T2Ds** | | | | |
|---|---|---|---|---|
| **Top 3 genes from pathway analysis** | | | | |
| **Probe** | **Gene Symbol** | **Gene Title** | | |
| 208900_s_at | TOP1 | topoisomerase (DNA) I | | |
| 216379_x_at | CD24 | CD24 antigen (smallcell lung carcinomacluster 4 antigen) | | |
| 1554343_a_at | STAP1 | signal transducingadaptor family member 1 | | |
| | | | | |

| **The mean expression of the top 3 genes in subgroups** | | | | |
|---|---|---|---|---|
| **Probe** | **Gene** | **Mean_NGT** | **Mean_IGT** | **Mean_T2D** |
| 208900_s_at | TOP1 | 868 | 1145 | 1418 |
| 216379_x_at | CD24 | 1767 | 1274 | 1194 |
| 1554343_a_at | STAP1 | 373 | 283 | 265 |

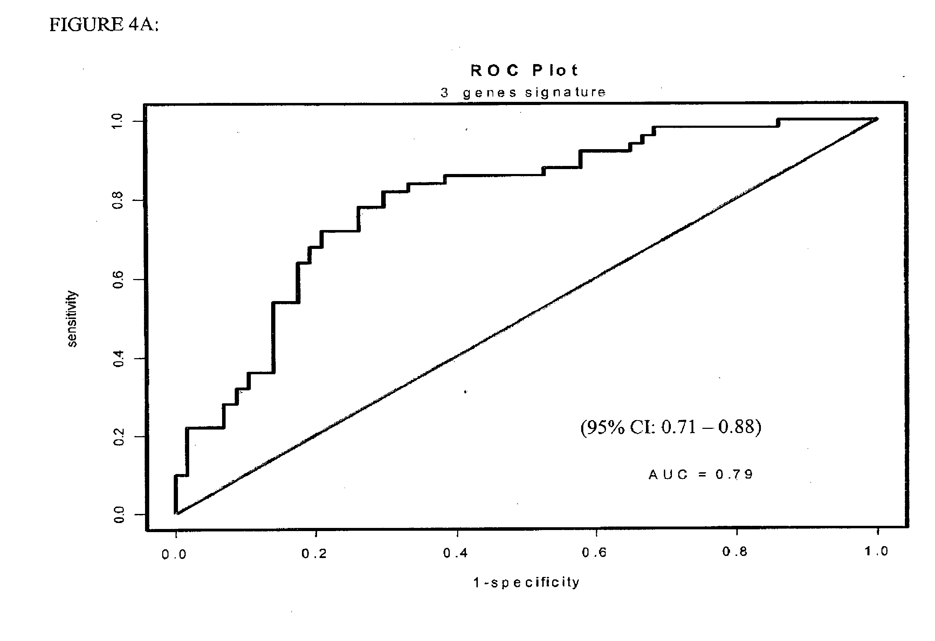

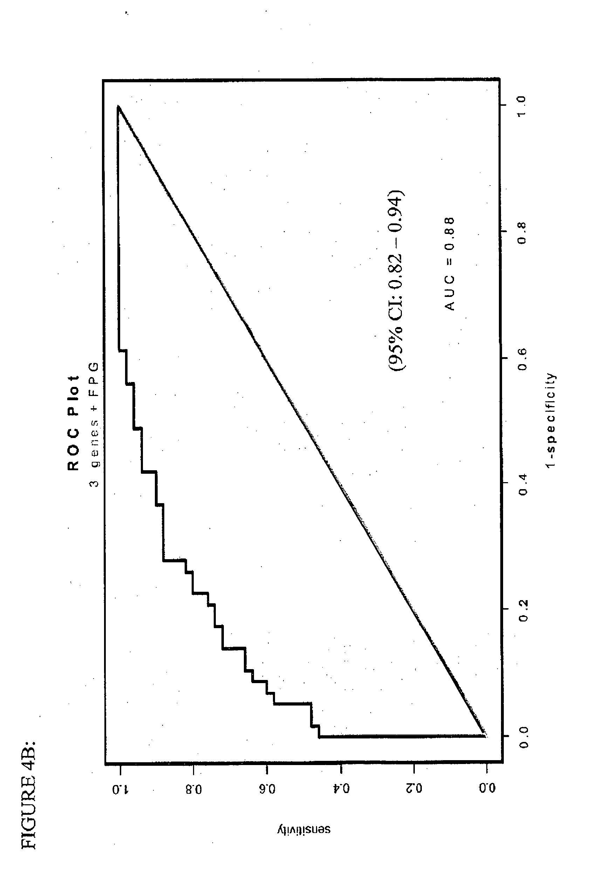

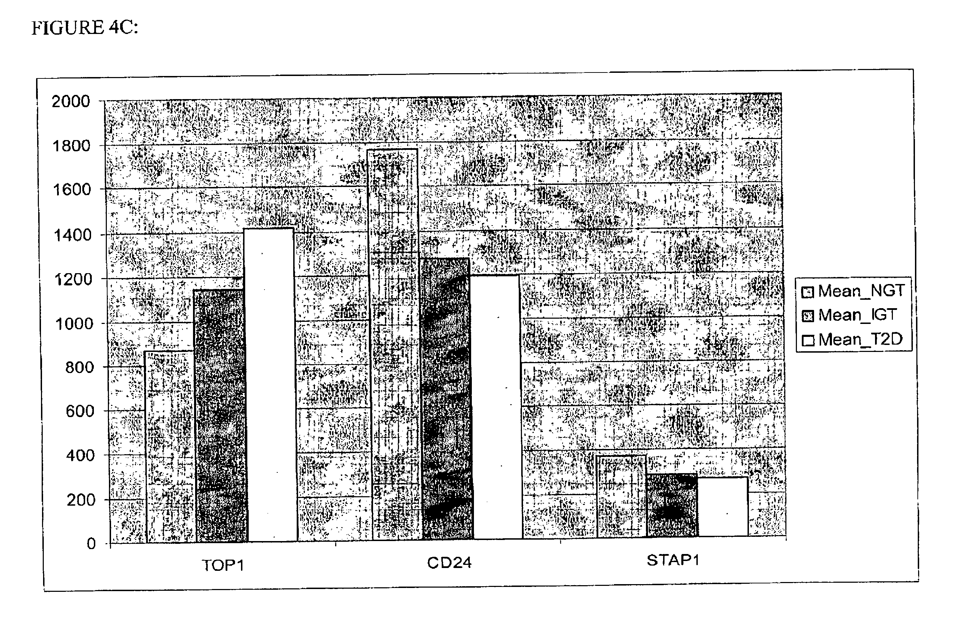

The ROC analysis of the 3-gene signature in the 107-patient cohort (FIG. 4A and 4B) demonstrates this signature can separate NGTs from IGTs/T2Ds. A histogram depicting the mean expression of the genes is shown in FIG. 4C.

To remove non-informative genes, only genes that had 10 or more presence calls in the cohort were retained. The 107-patient cohort was then divided into a 54-patient training set and a 53-patient test set. Based on OGTT classification, there are 28 NGTs, 17 IGTs and 9 T2Ds in the training set whereas there are 29 NGTs, 16 IGTs and 8 T2Ds in the test set. To identify genes that have differential expression between NGT and IGT + T2D patients, Significant Analysis of Microarray (SAM) program was performed. Genes were selected if the False Discovery Rate (FDR) is lower than 20%. As a result, 235 genes were selected. To further narrow down the gene list, genes with the fold-change larger than 1.5 between the two groups, and the average intensity of the gene in the dataset is larger than 200 were retained. As a result, 17 probe sets were obtained. Among them, 4 were probes representing hemoglobin gene. Considering that hemoglobin has extremely high expression in red blood cells, the 4 probes were removed to eliminate possible contamination. To determine the optimal number of genes as a signature, performance of combination of the top genes were examined from 2 to 13 in the training set. The result indicated that the top 10 genes gave the best performance based on the area under curve (AUC) (see Table 10).

**TABLE 10A: 10 gene expression in PBMC isolated from NGTs, IGTs and T2Ds**

| Probe | Symbol | Title |
|---|---|---|
| 239742 at | TULP4 | Tubby like protein 4 |
| 244450_at | AA741300 | Weakly similar to ALU8_HUMAN ALU SUBFAMILY SX SEQUENCE |
| 235216_at | ESCO1 | establishment of cohesion 1 homolog 1 |
| 201026_at | EIF5B | eukaryotic translation initiation factor 5B |
| 200727_s_at | ACTR2 | ARP2 actin-related protein 2 homolog |
| 211993_at | WNK1 | WNK lysine deficient protein kinase 1 |
| 205229_s_at | COCH | coagulation factor C homolog, cochlin |
| 201085_s_at | SON | SON DNA binding protein |
| 1557227_s_at | TPR | translocated promoter region (to activated MET oncogene) |
| 231798_at | NOG | Noggin |

**TABLE 10B: The mean expression of the top 10 genes in subgroups**

| **Probe** | **Gene** | **Mean**_**NGT** | **Mean**_**IGT** | **Mean_T2D** |
|---|---|---|---|---|
| 239742_at | TULP4 | 514 | 659 | 702 |
| 244450_at | AA741300 | 674 | 461 | 482 |
| 235216_at | ESCO1 | 199 | 262 | 351 |
| 201026_at | EIF5B | 330 | 440 | 500 |
| 200727_s_at | ACTR2 | 2153 | 2751 | 3590 |
| 211993_at | WNK1 | 397 | 505 | 625 |
| 205229_s_at | COCH | 330 | 231 | 250 |
| 201085_s_at | SON | 3300 | 4103 | 4900 |
| 1557227_s_at | TPR | 378 | 445 | 616 |
| 231798_at | NOG | 515 | 430 | 302 |

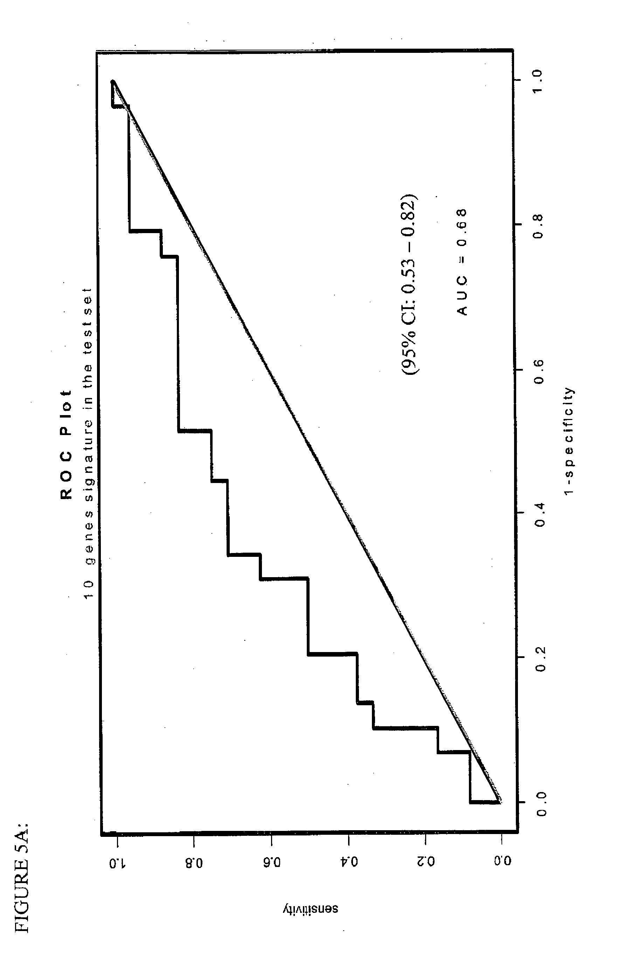

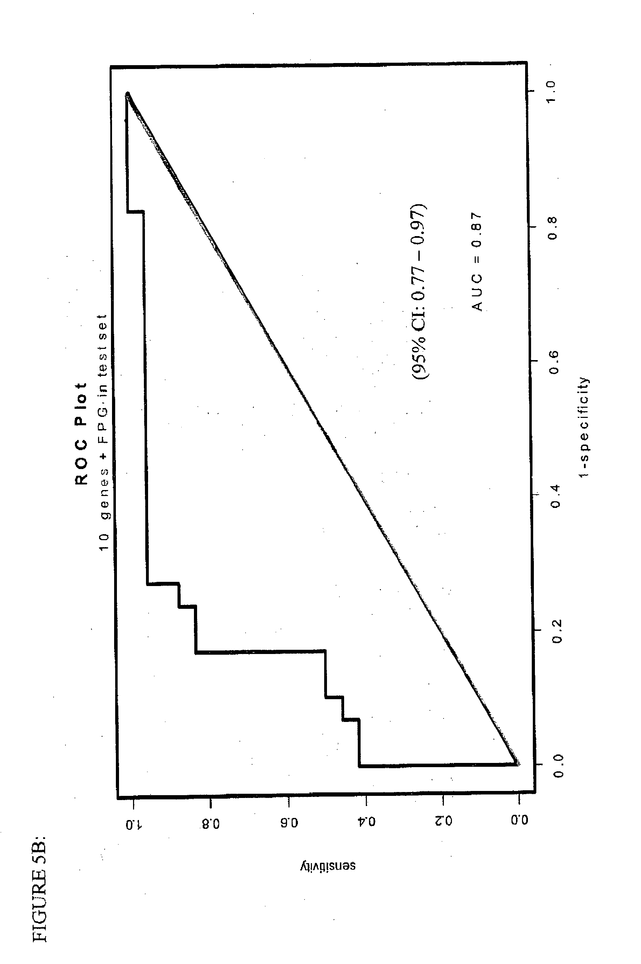

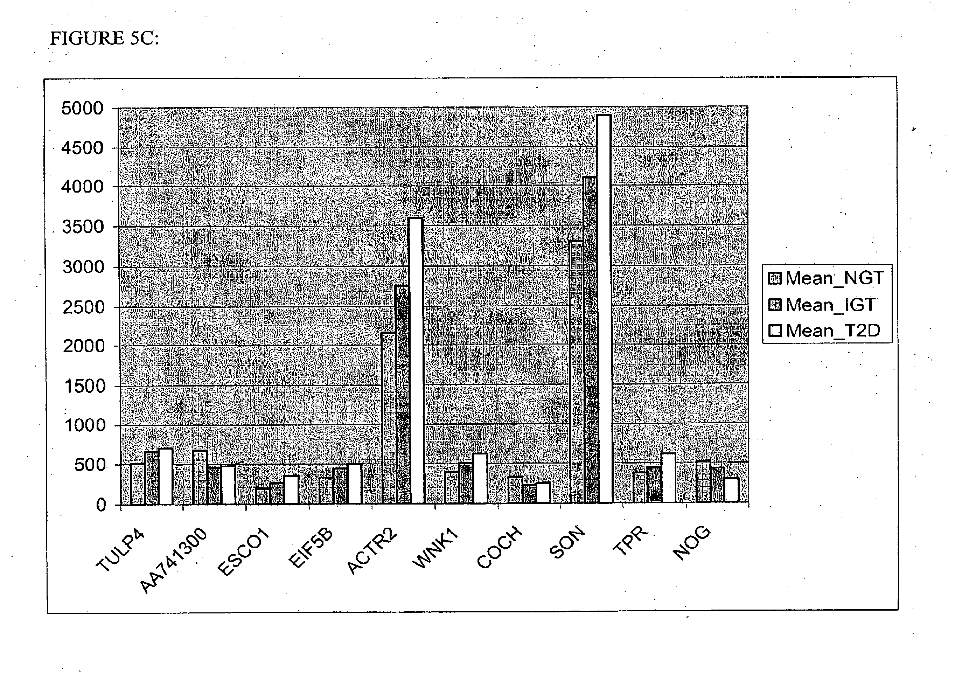

To further evaluate the gene signature, patient outcomes in the test set were determined. Prediction of pre-diabetes and diabetes using plasma fasting glucose (FPG) levels was also examined. To investigate the complementary effect between the gene signature and FPG levels, combination of these two predictors were used to predict the patient outcomes. A comparison of ROC analyses among using FPG, or 10-gene signature, or combination of FPG and 10-gene signature in the test set is depicted in FIG. 5. It demonstrates that the 10-gene signature can independently separate NTGs from IGTs/T2Ds, and the FPG and the 10-gene signature are complementary for better prediction (see FIGs. 5A and 5B). The mean expression signals of the 10 genes in the 107-patient cohort are shown in the table and bar chart in FIG. 5C.

The statistical analysis of the clinical data identified a 3 gene and 10 signature that are differentially expressed in NGTs and T2D.

In another embodiment, a diagnostic assay is described for the point-of-care classification of normal versus pre-diabetes/ diabetes or for the prediction of progression to pre-diabetes/ diabetes over a defined period time, e.g. from ½ to 2 years or from 2 to 5 years, or from 5 to 10 years or more.

Alternatively gene expression profiles are determined by detection of the protein encoded by the mRNA, for example using ELISA or proteomic array. All of these methods are well known in the art.

The disclosure herein also provides for a kit format which comprises a package unit having one or more reagents for the diagnosis of a diabetic disease state in a patient. The kit may also contain one or more of the following items: buffers, instructions, and positive or negative controls. Kits may include containers of reagents mixed together in suitable proportions for performing the methods described herein. Reagent containers preferably contain reagents in unit quantities that obviate measuring steps when performing the subject methods.

The kit may include sterile needles and tubes/ containers for the collection of a patient's blood. Collection tubes will typically contain certain additives e.g. heparin to inhibit blood coagulation

Kits may also contain reagents for the measurement of a gene signature expression profile in a patient's sample. As disclosed herein, gene signatures expression profiles may be measured by a variety of means known in the art including RT-PCR assays, oligonucleotide based assays using microchips or protein based assays such as ELISA assays.

In a preferred embodiment, gene signature expression profiles are measured by real-time RT-PCR.

In one embodiment of the application, the kit comprises primers of the amplification and detection of gene signature expression profiles in a patient's blood sample. Primers may have a sequence that is complementary to any one of the diabetes susceptibility genes as defined herein including TOP1, CD24, STAP1, TULP4, AA741300, ESCO1, EIF5B, ACTR2, WNK1, COCH, SON, TPR, NOG genes or any one of the genes listed in Tables 1 or 6.

Examples of primer sequences used for the real-time RT-PCR of diabetes susceptibility genes are disclosed in Tables 12B and 12C.

In a preferred embodiment, the kit reagents are designed to function with the 7500 Fast Dx Real-Time PCR Instrument by Applied Biosystems, which is a PCR-based technology that was approved by the FDA's Office of In Vitro Diagnostics (FDA-OIVD).

In yet another embodiment, the kit includes a microchip comprising an array of hybridization probes for the 3 gene (TOP1, CD24 and STAP1) or 10 gene (TULP4, AA741300, ESCO1, EIF5B, ACTR2, WNK1, COCH, SON, TPR and NOG) signatures. In another aspect, the microchips may further comprise an array of one or more hybrization probes for one or more of the genes listed in Tables 1 or 6.

In a preferred embodiment, the microchips are designed to function with Affymetrix GeneChipDx technology that can measure, in parallel, the gene expression of 1 to more than 55,000 mRNAs. FDA-OIVD this platform for use with the AmpliChip P450 product from Roche Molecular Diagnostics and the Pathwork Diagnostics Tissue of Origin test.

**TABLE 1**

| **Probe** | **Gene Symbol** | **Gene Title** |
|---|---|---|
| 218659_at | ASXL2 | additional sex combs like 2 (Drosophila) |
| 230528_s_at | MGC2752 | hypothetical protein MGC2752 |
| 211921_x_at | PTMA | prothymosin, alpha (gene sequence 28) *III* prothymosin, alpha (gene sequence 28) |
| 209102_s_at | HBP1 | HMG-box transcription factor 1 |
| 239946_at | KIAA0922 | KIAA0922 protein |
| 226741_at | TMEM85 | Transmembrane protein 85 |
| 239742_at | TULP4 | Tubby like protein 4 |
| 202844_s_at | RALBP1 | ralA binding protein 1 |
| 237768_x_at | TAF15 | TAF15 RNA polymerase II, TATA box binding protein (TBP)-associated factor, 68kDa |
| 202373_s_at | RAB3GAP2 | RAB3 GTPase activating protein subunit 2 (non-catalytic) |
| 223413_s_at | LYAR | hypothetical protein FLJ20425 |
| 222371_at | PIAS1 | Protein inhibitor of activated STAT, 1 |
| 244450_at | MAK | Male germ cell-associated kinase |
| 201024_x_at | EIF5B | eukaryotic translation initiation factor 5B |
| 202615_at | GNAQ | Guanine nucleotide binding protein (G protein), q polypeptide |
| 222621_at | DNAJC1 | DnaJ (Hsp40) homolog, subfamily C, member 1 |
| 212774_at | ZNF238 | zinc finger protein 238 |
| 238883_at | THRAP2 | Thyroid hormone receptor associated protein 2 |
| 223130_s_at | MYLIP | myosin regulatory light chain interacting protein |
| 225445_at | --- | Transcribed locus |
| 235601_at | MAP2K5 | Mitogen-activated protein kinase kinase 5 |
| 209258_s_at | CSPG6 | chondroitin sulfate proteoglycan 6 (bamacan) |
| 1557238_s_at | SETD5 | SET domain containing 5 |
| 202927_at | PIN1 | protein (peptidyl-prolyl cis/trans isomerase) NIMA-interacting 1 |
| 1568618_a_at | GALNT1 | UDP-N-acetyl-alpha-D-galactosamine:polypeptide N-acetylgalactosaminyltransferase 1 (GalNAc-T1) |
| 222417_s_at | SNX5 | sorting nexin 5 |
| 208836_at | ATP1B3 | ATPase, Na+/K+ transporting, beta 3 polypeptide |
| 202738_s_at | PHKB | phosphorylase kinase, beta |
| 224872_at | KIAA1463 | KIAA1463 protein |
| 235200_at | ZNF561 | Zinc finger protein 561 |
| 235216_a | ESCO1 | establishment of cohesion 1 homolog 1 (S. cerevisiae) |
| 201026_at | EIF5B | eukaryotic translation initiation factor 5B |
| 208095_s_at | SRP72 | signal recognition particle 72kDa |
| 244457_at | ITPR2 | Family with sequence similarity 20, member C |
| 216563_at | ANKRD12 | Ankyrin repeat domain 12 |
| 211983_x_at | ACTG1 | actin, gamma 1 |
| 227854_at | FANCL | Fanconi anemia, complementation group L |
| 1552343_s_at | PDE7A | phosphodiesterase 7A |
| 221548_s_at | ILKAP | integrin-linked kinase-associated serine/threonine phosphatase 2C |
| 215772_x_at | SUCLG2 | succinate-CoA ligase, GDP-forming, beta subunit |
| 229010_at | CBL | Cas-Br-M (murine) ecotropic retroviral transforming sequence |
| 226879_at | MGC15619 | hypothetical protein MGC15619 |
| 1556451_at | BACH2 | BTB and CNC homology 1, basic leucine zipper transcription factor 2 |
| 225490_at | ARID2 | AT rich interactive domain 2 (ARID, RFX-like) |
| 214055_x_at | BAT2D1 | BAT2 domain containing 1 |
| 32069_at | N4BP1 | Nedd4 binding protein 1 |
| 235457_at | MAML2 | mastermind-like 2 (Drosophila) |
| 217985_s_at | BAZ1A | bromodomain adjacent to zinc finger domain, 1A |
| 229399_at | C10orf118 | chromosome 10 open reading frame 118 |
| 208994_s_at | PPIG | peptidyl-prolyl isomerase G (cyclophilin G) |
| 202656_s_at | SERTAD2 | SERTA domain containing 2 |
| 241917_at | FCHSD2 | FCH and double SH3 domains 2 |
| 238807_at | ANKRD46 | Ankyrin repeat domain 46 |
| 204415_at | G1P3 | interferon, alpha-inducible protein (clone IFI-6-16) |
| 240176_at | LOC391426 | Similar to ENSANGP00000004103 |
| 233284_at | --- | --- |
| 232583_at | --- | --- |
| 200772_x_at | PTMA | prothymosin, alpha (gene sequence 28) |
| 239721_at | UBE2H | Ubiquitin-conjugating enzyme E2H (UBC8 homolog, yeast) |
| 218607_5_at | SDAD1 | SDA1 domain containing 1 |
| 204160_s_at | ENPP4 | ectonucleotide pyrophosphatase/phosphodiesterase 4 (putative function) |
| 243303_at | ECHDC1 | Enoyl Coenzyme A hydratase domain containing 1 |
| 225266_at | ZNF652 | Zinc finger protein 652 |
| 220072_at | CSPP1 | centrosome and spindle pole associated protein 1 |
| 234196_at | TMCC3 | Transmembrane and coiled-coil domain family 3 |
| 222616_s_at | USP16 | ubiquitin specific peptidase 16 |
| 201274_at | PSMA5 | proteasome (prosome, macropain) subunit, alpha type, 5 |
| 238714_at | RAB12 | RAB12, member RAS oncogene family |
| 204563_at | SELL | selectin L (lymphocyte adhesion molecule 1) |
| 1557239_at | BBX | Bobby sox homolog (Drosophila) |
| 232510_s_at | DPP3 | dipeptidylpeptidase 3 |
| 235653_s_at | THAP6 | THAP domain containing 6 |
| 200727_s_at | ACTR2 | ARP2 actin-related protein 2 homolog (yeast) |
| 221564_at | HRMT1L1 | HMT1 hnRNP methyltransferase-like 1 (S. cerevisiae) |
| 211993_at | WNK1 | WNK lysine deficient protein kinase 1 // WNK lysine deficient protein kinase 1 |
| 201114_x_at | PSMA7 | proteasome (prosome, macropain) subunit, alpha type, 7 |
| 233089_at | QRSL1 | glutaminyl-tRNA synthase (glutamine-hydrolyzing)-like 1 |
| 212991_at | FBXO9 | F-box protein 9 |
| 227770_at | VPS4A | Vacuolar protein sorting 4A (yeast) |
| 222111_at | FAM63B | Family with sequence similarity 63, member B |
| 1558604_a_at | --- | MRNA; clone CD 43T7 |
| 205229_s_at | COCH | coagulation factor C homolog, cochlin (Limulus polyphemus) |
| 219130_at | FLJ10287 | hypothetical protein FLJ10287 |
| 241262_at | --- | --- |
| 202412_s_at | USP1 | ubiquitin specific peptidase 1 |
| 225092_at | RABEP1 | rabaptin, RAB GTPase binding effector protein 1 |
| 200905_x_at | HLA-E | major histocompatibility complex, class I, E |
| 201010_s_at | TXNIP | thioredoxin interacting protein |
| 221607_x_at | ACTG1 | actin, gamma 1 |
| 201085_s_at | SON | SON DNA binding protein |
| 214723_x_at | KIAA1641 | KIAA1641 |
| 201565_s_at | ID2 | inhibitor of DNA binding 2, dominant negative helix-loop-helix protein |
| 201861_s_at | LRRFIP1 | leucine rich repeat (in FLII) interacting protein 1 |
| 207785_s_at | RBPSUH | recombining binding protein suppressor of hairless (Drosophila) |
| 230415_at | --- | --- |
| 236620_at | RIF1 | RAP1 interacting factor homolog (yeast) |
| 206363_at | MAF | v-maf musculoaponeurotic fibrosarcoma oncogene homolog (avian) |
| 1558748_at | NAPE-PLD | N-acyl-phosphatidylethanolamine-hydrolyzing phospholipase D |
| 223101_s_at | ARPC5L | actin related protein 2/3 complex, subunit 5-like |
| 236370_at | SMURF1 | SMAD specific E3 ubiquitin protein ligase 1 |
| 200702_s_at | DDX24 | DEAD (Asp-Glu-Ala-Asp) box polypeptide 24 |
| 1557227_s_at | TPR | translocated promoter region (to activated MET oncogene) |
| 220934_s_at | MGC3196 | hypothetical protein MGC3196 |
| 233333_x_at | AVIL | advillin |
| 231798_at | NOG | Noggin |
| 228986_at | OSBPL8 | oxysterol binding protein-like 8 |
| 241786_at | PPP3R1 | Protein phosphatase 3 (formerly 2B), regulatory subunit B, 19kDa, alpha isoform (calcineurin B, type I) |
| 212227_x_at | EIF1 | eukaryotic translation initiation factor 1 |
| 222471_s_at | KCMF1 | potassium channel modulatory factor 1 |
| 203580_s_at | SLC7A6 | solute carrier family 7 (cationic amino acid transporter, y+ system), member 6 |
| 208900_s_at | TOP1 | topoisomerase (DNA) I |
| 240070_at | FLJ39873 | hypothetical protein FLJ39873 |
| 213305_s_at | PPP2R5C | protein phosphatase 2, regulatory subunit B (B56), gamma isoform |
| 229470_at | --- | CDNA FLJ27196 fis, clone SYN02831 |
| 204048_s_at | PHACTR2 | phosphatase and actin regulator 2 |
| 1561690_at | --- | CDNA clone IMAGE:5303966 |
| 1556728_at | --- | CDNA FLJ43665 fis, clone SYNOV4006327 |
| 212027_at | RBM25 | RNA binding motif protein 25 |
| 210218_s_at | SP100 | nuclear antigen Sp100 |
| 232356_at | --- | CDNA FLJ13539 fis, clone PLACE1006640 |
| 241891_at | DOCK8 | Dedicator of cytokinesis 8 |
| 235925_at | LOC440282 | Hypothetical protein LOC145783 |
| 211745_x_at | HBA1 | hemoglobin, alpha 1 /// hemoglobin, alpha 1 |
| 240452_at | GSPT1 | G1 to S phase transition 1 |
| 212669_at | CAMK2G | calcium/calmodulin-dependent protein kinase (CaM kinase) II gamma |
| 209791_at | PADI2 | peptidyl arginine deiminase, type II |
| 221952_x_at | TRMT5 | TRM5 tRNA methyltransferase 5 homolog (S. cerevisiae) |
| 226942_at | PHF20L1 | PHD finger protein 20-like 1 |
| 203939_at | NT5E | 5'-nucleotidase, ecto (CD73) |
| 208705_s_at | EIF5 | eukaryotic translation initiation factor 5 |
| 1557718_at | PPP2R5C | protein phosphatase 2, regulatory subunit B (B56), gamma isoform |
| 212251_at | MTDH | metadherin |
| 226384_at | PPAPDC1 B | phosphatidic acid phosphatase type 2 domain containing 1B |
| 212487_at | KIAA0553 | KIAA0553 protein |
| 227402_s_at | C8orf53 | chromosome 8 open reading frame 53 |
| 221875_x_at | HLA-F | major histocompatibility complex, class I, F |
| 225506_at | KIAA1468 | KIAA1468 |
| 201730_s_at | TPR | translocated promoter region (to activated MET oncogene) |
| 235645_at | ESCO1 | establishment of cohesion 1 homolog 1 (S. cerevisiae) |
| 208993_s_at | PPIG | peptidyl-prolyl isomerase G (cyclophilin G) |
| 233690_at | C21orf96 | Chromosome 21 open reading frame 96 |
| 221798_x_at | RPS2 | Ribosomal protein S2 |
| 1569898_a_at | --- | CDNA FLJ32047 fis, clone NTONG2001137 |
| 202368_s_at | TRAM2 | translocation associated membrane protein 2 |
| 215128_at | --- | --- |
| 230761_at | USP7 | Unknown protein |
| 243_g_at | MAP4 | microtubule-associated protein 4 |
| 223081_at | PHF23 | PHD finger protein 23 |
| 224736_at | CCAR1 | cell division cycle and apoptosis regulator 1 |
| 236962_at | PTBP2 | Polypyrimidine tract binding protein 2 |
| 225893_at | --- | MRNA; cDNA DKFZp686D04119 (from clone DKFZp686D04119) |
| 244414_at | MAML2 | Mastermind-like 2 (Drosophila) |
| 221234_s_at | BACH2 | BTB and CNC homology 1, basic leucine zipper transcription factor 2 /// BTB and CNC homology 1, basic zipper transcription factor 2 |
| 218135_at | PTX1 | PTX1 protein |
| 229353_s_at | NUCKS1 | nuclear casein kinase and cyclin-dependent kinase substrate 1 |
| 228408_s_at | SDAD1 | SDA1 domain containing 1 |
| 234723_x_at | --- | --- |
| 212130_x_at | EIF1 | eukaryotic translation initiation factor 1 |
| 232565_at | RAB6IP2 | RAB6 interacting protein 2 |
| 210479_s_at | RORA | RAR-related orphan receptor A |
| 226320_at | THOC4 | THO complex 4 |
| 208859_s_at | ATRX | alpha thalassemia/mental retardation syndrome X-linked (RAD54 homolog, S. cerevisiae) |
| 238645_at | VIL2 | Villin 2 (ezrin) |
| 243578_at | --- | Transcribed locus |
| 202868_s_at | POP4 | processing of precursor 4, ribonuclease P/MRP subunit (S. cerevisiae) |
| 224585_x_at | ACTG1 | actin, gamma 1 |
| 221768_at | SFPQ | Splicing factor proline/glutamine-rich (polypyrimidine tract binding protein associated) |
| 1557459_at | SNF1LK2 | SNF1-like kinase 2 |
| 225583_at | UXS1 | UDP-glucuronate decarboxylase 1 |
| 225125_at | TMEM32 | transmembrane protein 32 |
| 202408_s_at | PRPF31 | PRP31 pre-mRNA processing factor 31 homolog (yeast) |
| 236355_s_at | LOC439993 | LOC439993 |
| 209458_x_at | HBA1 /// HBA2 | hemoglobin, alpha 1 /// hemoglobin, alpha 1 /// hemoglobin, alpha 2 /// hemoglobin, alpha 2 |
| 211948_x_at | BAT2D1 | BAT2 domain containing 1 |
| 203682_s_at | IVD | isovaleryl Coenzyme A dehydrogenase |
| 203184_at | FBN2 | fibrillin 2 (congenital contractural arachnodactyly) |
| 1560082_at | NOL10 | Nucleolar protein 10 |
| 212794_s_at | KIAA1033 | KIAA1033 |
| 226159_at | LOC285636 | hypothetical protein LOC285636 |
| 225276_at | GSPT1 | G1 to S phase transition 1 |
| 205859_at | LY86 | lymphocyte antigen 86 |
| 200977_s_at | TAX1BP1 | Tax1 (human T-cell leukemia virus type I) binding protein 1 |
| 239418T_x_at | ENTPD1 | Ectonucleoside triphosphate diphosphohydrolase 1 |
| 208638_at | PDIA6 | protein disulfide isomerase family A, member 6 |
| 203228_at | PAFAH1B3 | platelet-activating factor acetylhydrolase, isoform Ib, gamma subunit 29kDa |
| 208812_x_at | HLA-C | major histocompatibility complex, class I, C |
| 220924_s_at | SLC38A2 | solute carrier family 38, member 2 |
| 235705_at | --- | --- |
| 208974_x_at | KPNB1 | karyopherin (importin) beta 1 |
| 201854_s_at | ASCIZ | ATM/ATR-Substrate Chk2-Interacting Zn2+-finger protein |
| 209116_x_at | HBB | hemoglobin, beta /// hemoglobin, beta |
| 218150_at | ARL5 | ADP-ribosylation factor-like 5 |
| 208042_at | AGGF1 | angiogenic factor with G patch and FHA domains 1 |
| 226718_at | AMIGO1 | adhesion molecule with Ig-like domain 1 |
| 235328_at | CCDC41 | Coiled-coil domain containing 41 |
| 225609_at | GSR | glutathione reductase |
| 242972_at | --- | CDNA FLJ46556 fis, clone THYMU3039807 |
| 239811_at | MLL5 | Myeloid/lymphoid or mixed-lineage leukemia 5 (trithorax homolog, Drosophila) |
| 201027_s_at | EIF5B | eukaryotic translation initiation factor 5B |
| 233742_at | MGC2654 | LP8272 |
| 1556323_at | CUGBP2 | CUG triplet repeat, RNA binding protein 2 |
| 202926_at | NAG | neuroblastoma-amplified protein |
| 220966_x_at | ARPC5L | actin related protein 2/3 complex, subunit 5-like /// actin related protein 2/3 complex, subunit 5-like |
| 1552302_at | MGC20235 | hypothetical protein MGC20235 |
| 238787_at | --- | Transcribed locus |
| 213505_s_at | SFRS14 | splicing factor, arginine/serine-rich 14 |
| 1555920_at | CBX3 | Chromobox homolog 3 (HP1 gamma homolog, Drosophila) |
| 207186_s_at | FALZ | fetal Alzheimer antigen |
| 210426_x_at | RORA | RAR-related orphan receptor A |
| 1559993_at | --- | --- |
| 201602_s_at | PPP1R12A | protein phosphatase 1, regulatory (inhibitor) subunit 12A |
| 216088_s_at | PSMA7 | proteasome (prosome, macropain) subunit, alpha type, 7 |
| 236254_at | VPS13B | vacuolar protein sorting 13B (yeast) |
| 204731_at | TGFBR3 | transforming growth factor, beta receptor III (betaglycan, 300kDa) |
| 202269_x_at | GBP1 | guanylate binding protein 1, interferon-inducible, 67kDa /// guanylate binding protein 1, interferon-inducible, 67kDa |
| 216981_x_at | SPN | sialophorin (gpL115, leukosialin, CD43) |
| 212007_at | UBXD2 | UBX domain containing 2 |
| 217755_at | HN1 | hematological and neurological expressed 1 |
| 213940_s_at | FNBP1 | formin binding protein 1 |
| 201831_s_at | VDP | vesicle docking protein p115 |
| 225041_at | HSMPP8 | M-phase phosphoprotein, mpp8 |
| 1552584_at | IL12RB1 | interleukin 12 receptor, beta 1 |
| 206133_at | BIRC4BP | XIAP associated factor-1 |
| 229625_at | GBP5 | Guanylate binding protein 5 |
| 206500_s_at | C14orf106 | chromosome 14 open reading frame 106 |
| 201881_s_at | ARIH1 | ariadne homolog, ubiquitin-conjugating enzyme E2 binding protein, 1 (Drosophila) |
| 202323_s_at | ACBD3 | acyl-Coenzyme A binding domain containing 3 |
| 204021_s_a.t | PURA | purine-rich element binding protein A |
| 215313_x_at | HLA-A | major histocompatibility complex, class I, A |
| 207966_s_at | GLG1 | golgi apparatus protein 1 |
| 235461_at | FLJ20032 | hypothetical protein FLJ20032 |
| 223983_s_at | C19orf12 | chromosome 19 open reading frame 12 |
| 202021_x_at | EIF1 | eukaryotic translation initiation factor 1 |
| 231577_s_at | GBP1 | guanylate binding protein 1, interferon-inducible, 67kDa |
| 218927_s_at | CHST12 | carbohydrate (chondroitin 4) sulfotransferase 12 |

**TABLE 11**

| | **SELECTED GENES** |
|---|---|
| **Gene** | **Description (functions, domains)** |
| **TCF7L2** | The TCL7L2 gene product is a high mobility group (HMG) box-containing transcription factor implicated in blood glucose homeostasis. High mobility group (HMG or HMGB) proteins are a family of relatively low molecular weight non-histone components in chromatin. HMG1 (also called HMG-T in fish) and HMG2 are two highly related proteins that bind single-stranded DNA preferentially and unwind double-stranded DNA. Although they have no sequence specificity, they have a high affinity for bent or distorted DNA, and bend linear DNA. HMG1 and HMG2 contain two DNA-binding HMG-box domains (A and B) that show structural and functional differences, and have a long acidic C-terminal domain rich in aspartic and glutamic acid residues. The acidic tail modulates the affinity of the tandem HMG boxes in HMG1 and 2 for a variety of DNA targets. HMG1 and 2 appear to play important architectural roles in the assembly of nucleoprotein complexes in a variety of biological processes, for example V(D)J recombination, the initiation of transcription, and DNA repair. |
| **CLC** | The protein encoded by this gene is a lysophosphollpase expressed in eosinophils and basophils. It hydrolyzes lysophosphatidylcholine to glycerophosphocholine and a free fatty acid. This protein may possess carbohydrate or IgE-binding activities. It is both structurally and functionally related to the galectin family of beta-galactoside binding proteins. It may be associated with inflammation and some myeloid leukemias. Galectins (previously S-lectins) bind exclusively beta-galactosides like lactose. They do not require metal ions for activity. Galectins are found predominantly, but not exclusively in mammals. Their function is unclear. They are developmentally regulated and may be involved In differentiation, cellular regulation and tissue construction. |
| **CDKN1C** | protein encoded by this gene is a tight-binding, strong inhibitor of several G1 cyclin/Cdk complexes and a negative regulator of cell proliferation. Mutations in this gene are implicated in sporadic cancers and Beckwith-Wiedemann syndorome, suggesting that this gene is a tumor suppressor candldate. Three transcript variants encoding two different isoforms have been found for this gene. |

| | **3 GENE SIGNATURE** |
|---|---|
| **Gene** | **Description (functions, domains)** |
| **TOP1** | This gene encodes a DNA topolsomerase, an enzyme that controls and alters the topologic states of DNA during transcription. DNA topoisomerase: the number of topological links between two DNA strands (i.e. change the number of superhelical turns) by catalysing transient single- or double-str; breaks, crossing the strands through one another, then resealing the breaks. These enzymes have several functions: to remove DNA supercoils dui transcription and DNA replication; for strand breakage during recombination; for chromosome condensation: and to disentangle intertwined DNA durit mitosis. DNA topoisomerases are divided into two classes: type I enzymes break single-strand DNA, and type II enzymes break double-strand DNA. Type I topoisomerases are ATP-independent enzymes (except for reverse gyrase), and can be subdivided according to their structure and reaction mechanisms: type IA (bacterial and archaeal topoisomerase I, topoisomerase III and reverse gyrase) and type IB (eukaryotic topoisomerase I and topoisomerase V). These enzymes are primarily responsible for relaxing positively and/or negatively supercoiled DNA, except for reverse gyrase, which introduce positive supercoils into DNA. |
| | The crystal structures of human topoisomerase I comprising the core and carboxyl-terminal domains in covalent and noncovalent complexes with 22-ba DNA duplexes reveal an enzyme that "clamps" around essentially B-form DNA. The core domain and the first eight residues of the carboxyl-terminal do of the enzyme, including the active-site nucleophile tyrosine-723, share significant structural similarity with the bacteriophage family of DNA integrases. A binding mode for the anticancer drug camptothecin has been proposed on the basis of chemical and biochemical Information combined with the three-dimensional structures of topoisomerase I-DNA complexes. |
| **CD24** | This gene encodes a sialoglycoprotein that Is expressed on mature granulocytes and in many B cells. The encoded protein is anchored via a glycosyl phosphatidylinositol (GPI) link to the cell surface. |
| **STAP1** | The protein encoded by this gene functions as a docking protein acting downstream of Tec tyrosine kinase in B cell antigen receptor signaling. The protein directly phosphorylated by Tec in vitro where it participates in a positive feedback loop, increasing Tec activity. |

| | **10 GENE SIGNATURE** |
|---|---|
| **Gene** | **Description (functions, domains)** |
| **TULP4** | Tubby like protein 4 contains WD40 and SOCS domains. WD-40 repeats (also known as WD or beta-transducin repeats) are short ∼40 amino acid motifs, often terminating in a Trp-Asp (W-D) dipeptide. WD-containing proteins have 4 to 16 repeating units, all of which are thought to form a circularised beta-propeller structure. WD-repeat proteins are a large family found in all eukaryotes and are implicated in a variety of functions ranging from signal transduction and transcription regulation to cell cycle control and apoptosis. The underlying common function of all WD-repeat proteins is coordinating multi-protein complex assemblies, where the repeating units serve as a rigid scaffold for protein interactions. The specificity of the proteins is determined by the sequences outside the repeats themselves. Examples of such complexes are G proteins (beta subunit is a beta-propeller), TAFII transcription factor, and E3 ubiquitin ligase. |
| | The SOCS box was first identifed in SH2-domain-containing proteins of the suppressor of cytokines signaling (SOCS) family but was later also found In: the WSB (WD-40-repeat-containing proteins with a SOCS box) family, the SSB (SPRY domain-containing proteins with a SOCS box) family, the ASB (ankyrin-repeat-containing proteins with a SOCS box) family, and ras and ras-like GTPases. The SOCS box found in these proteins is an about 50 amino acid carboxy-terminal domain composed of two blocks of well-conserved residues separated by between 2 and 10 nonconserved residues. The C-terminal conserved region is an L/P-rich sequence of unknown function, whereas the N-terminal conserved region is a consensus BC box, which binds to the Elongin BC complex. It has been proposed that this association could couple bound proteins to the ubiquitination or proteasomal compartments. |
| **AA741300** | Unknown protein (New protein) |
| **ESCO1** | establishment of cohesion 1 homolog 1 (ESCO1) belongs to a conserved family of acetyltransferases involved In sister chromatid cohesion. |
| **EIF5B** | Accurate initiation of translation in eukaryotes is complex and requires many factors, some of which are composed of multiple subunits. The process is simpler in prokaryotes which have only three initiation factors (IF1, IF2, IF3). Two of these factors are conserved in eukaryotes: the homolog of IF1 is eIF1A and the homolog of IF2 is eIF5B. This gene encodes eIF5B. Factors eIF1A and eIF5B interact on the ribosome along with other initiation factors and GTP to position the initiation methionine tRNA on the start codon of the mRNA so that translation initiates accurately. |

| **Gene** | **Description (functions, domains)** |
|---|---|
| **ACTR2** | ARP2 actin-related protein 2 homolog (ACTR2) Is known to be a major constituent of the ARP2/3 complex. This complex is located at the cell surface and is essential to cell shape and motility through lamellipodial actin assembly and protrusion. Two transcript variants encoding different isoforms have been found for this gene. |
| **WNK1** | The WNK1 gene encodes a cytoplasmic serine-threonine kinase expressed in distal nephron. Protein kinases are a group of enzymes that possess a catalytic subunit which transfers the gamma phosphate from nucleotide triphosphates (often ATP) to one or more amino acid residues in a protein substrate side chain, resulting in a conformational change affecting protein function. The enzymes fall into two broad classes, characterised with respect to substrate specificity: serine/threonine specific and tyrosine specific. |
| | Protein kinase function has been evolutionarily conserved from Escherichia coli to Homo sapiens. Protein kinases play a role in a mulititude of cellular processes, including division, proliferation, apoptosis, and differentiation. Phosphorylation usually results in a functional change of the target protein by changing enzyme activity, cellular location, or association with other proteins. |
| | The catalytic subunits of protein kinases are highly conserved, and several structures have been solved, leading to large screens to develop kinase-specific inhibitors for the treatments of a number of diseases. Eukaryotic protein kinases are enzymes that belong to a very extensive family of proteins which share a conserved catalytic core common with both serine/threonine and tyrosine protein kinases. There are a number of conserved regions in the catalytic domain of protein kinases. In the N-torminal extremity of the catalytic domain there is a glycine-rich stretch of residues in the vicinity of a lysine residue, which has been shown to be involved in ATP binding. In the central part of the catalytic domain there is a conserved aspartic acid residue which Is important for the catalytic activity of the enzyme. |
| **COCH** | The protein encoded by this gene is highly conserved in human, mouse, and chicken, showing 94% and 79% amino acid identity of human to mouse and chicken sequences, respectively. Hybridization to this gene was detected in spindle-shaped cells located along nerve fibers between the auditory ganglion and sensory epithelium. These cells accompany neurites at the habenula perforata, the opening through which neurites extend to innervate hair cells. This and the pattern of expression of this gene in chicken inner ear paralleled the histologic findings of acidophilic deposits, consistent with mucopolysaccharide ground substance, in temporal bones from DFNA9 (autosomal dominant nonsyndromic sensorineural deafness 9) patients. Mutations that cause DFNA9 have been reported in this gene. Alternative splicing results in multiple transcript variants encoding the same protein. Additional splice variants encoding distinct isoforms have been described but their biological validities have not been demonstrated. The protein contains a VWA domains in extracellular eukaryotic proteins mediate adhesion via metal ion-dependent adhesion sites (MIDAS). Intracellular VWA domains and homologues In prokaryotes have recently been identified. The proposed VWA domains in integrin beta subunits have recently been substantiated using sequence-based methods. |
| **SON** | The protein encoded by this gene binds to a specific DNA sequence upstream of the upstream regulatory sequence of the core promoter and second enhancer of human hepatitis B virus (HBV). Through this binding, it represses HBV core promoter activity, transcription of HBV genes, and production of HBV virions. The protein shows sequence similarities with other DNA-binding structural proteins such as gallin, oncoproteins of the MYC family, and the oncoprotein MOS. It may also be involved In protecting cells from apoptosis and in pre-mRNA splicing. Several transcript variants encoding different isoforms have been described for this gene, but the full-length nature of only two of them has been determined. Members of this family belong to the collagen superfamily. Collagens are generally extracellular structural proteins Involved In formation of connective tissue structure. The sequence is predominantly repeats of the G-X-Y and the polypeptide chains form a triple helix. The first position of the repeat is glycine, the second and third positions can be any residue but are frequently proline and hydroxyproline. Collagens are post-translationally modified by proline hydroxylase to form the hydroxyproline residues. Defective hydroxylation is the cause of scurvy. Some members of the collagen superfamily are not involved in connective tissue structure but share the same triple helical structure. |
| **TPR** | This gene encodes a large coiled-coil protein that forms intranuclear filaments attached to the Inner surface of nuclear pore complexes (NPCs). The protein directly interacts with several components of the NPC. It Is required for the nuclear export of mRNAs and some proteins. Oncogenic fusions of the 5' end of this gene with several different kinase genes occur in some neoplasias. Intermediate filaments (IF) are proteins which are primordial components of the cytoskeleton and the nuclear envelope. They generally form filamentous structures 8 to 14 nm wide. IF proteins are members of a very large multigene family of proteins which has been subdivided in five major subgroups: Type I: Acidic cytokeratins. Type II: Basic cytokeratins. Type III: Vimentin, desmin, glial fibrillary acidic protein (GFAP), peripherin, and plasticin. Type IV: Neurofilaments L, H and M, alpha-Internexin and nestin. Type V: Nuclear lamins A, B1, B2 and C. All IF proteins are structurally similar In that they consist of: a central rod domain comprising some 300 to 350 residues which is arranged in colled-colled alpha-helices, with at least two short characteristic interruptions; a N-terminal non-helical domain (head) of variable length: and a C-terminal domain (tail) which is also non-helical, and which shows extreme length variation between different IF proteins. While IF proteins are evolutionary and structurally related, they have limited sequence homologies except in several regions of the rod domain. This entry represents the central rod domain found in IF proteins. |
| **NOG** | The secreted polypeptide, encoded by this gene, binds and inactivates members of the transforming growth factor-beta (TGF-beta) superfamily signaling proteins, such as bone morphogenetic protein-4 (BMP4). By diffusing through extracellular matrices more efficiently than members of the TGF-beta superfamily, this protein may have a principal role in creating morphogenic gradients. The protein appears to have pleiotropic effect, both early in development as well as In later stages. It was originally isolated from Xenopus based on its ability to restore normal dorsal-ventral body axis in embryos that had been artificiallyventralized by UV treatment. The results of the mouse knockout of the ortholog suggest that it is involved In numerous developmental processes, such as neural tube fusion and joint formation. Recently, several dominant human NOG mutations in unrelated families with proximal symphalangism (SYM1) and multiple synostoses syndrome (SYNS1) were identified; both SYM1 and SYNS1 have multiple joint fusion as their principal feature, and map to the same region (17q22) as this gene. All of these mutations altered evolutionarily conserved amino acid residues. The amino acid sequence of this human gene is highly homologous to that of Xenopus, rat and mouse. This family consists of the eukaryotic Noggin proteins. Noggin is a glycoprotein that binds bone morphogenetic proteins (BMPs) selectively and, when added to osteoblasts, it opposes the effects of BMPs. It has been found that noggin arrests the differentiation of stromal cells, preventing cellular maturation. |

**TABLE 12A**

| **Gene symbol** | **GenBank Accession Number** | | **3-gene signature** | |
|---|---|---|---|---|
| | | | **Gene symbol** | **GenBank Accession Number** |
| CDKN1C | gi\|169790897\|ref\|NM_000076.2\| | | TOP1 | NM_003286 |
| TCF7L2 | gi\|170014695\|ref\|NM_030756.3 | | CD24 | NM_013230 |
| | | | STAP1 | NM_012108 |
| CLC | gi\|20357558\|ref\|NM_001828.4\| | | | |
| | | | **10-gene signature** | |
| WFS1 | NM_006005 | | **Gene symbol** | **GenBank Accession Number** |
| TSPAN8 | NM_004616 | | | |
| THADA | NM_022065 | | TULP4 | NM_020245 |
| TCF7L2 | NM_030756 | | AA741300 | AA741300 |
| SLC30A8 | NM_173851 | | ESCO1 | NM_052911 |
| PPARG | NM_138712 | | EIF5B | NM_015904 |
| NOTCH2 | NM_024408 | | ACTR2 | NM_001005386 |
| LGR5 | NM_003667 | | WNK1 | NM_018979 |
| KCNJ11 | NM_000525 | | COCH | NM_004086 |
| JAZF1 | NM_175061 | | SON | NM_138927 |
| IGF2BP2 | NM_001007225 | | TPR | NM_003292 |
| HHEX-IDE | NM_002729, NM_004969 | | NOG | NM_005450 |
| FTO | NM_001080432 | | | |
| CDKN2B | NM_078487 | | | |
| CDKN2A | NM_058195 | | | |
| CDC123 | NM_006023 | | | |
| CAMK1 D | NM_153498 | | | |
| ADAMTS9 | NM_182920 | | | |

**Table 12 B**

| **3-gene signature** | | | | |
|---|---|---|---|---|
| **Gene symbol** | **Accession** | **Upper primer Sequence** | **Probe Sequence** | **Lower primer Sequence** |
| TOP1 | NM_003286 | CCCTGTACTTCATCGACAAGC | AGCAGCAGCCCACAGTGT | AGAGCAGGCAATGAAAAGGAGGAAG |
| CD24 | NM_013230 | GCCAGGGCAATGATGAATG | CTCAATATGGATAATCAAGAGTTGCT | TCTACCCCCAGATCCAAGCAGCCT |
| STAP1 | NM_012108 | TGAAAAGAACTGTGCGAAATTC | CACTTTCTGTGTTCTCTGTCTTCAG | CCTTGTTTTGCCGAAAGAGGAAGTACA |
| | | | | |
| STAP1-331F22 | | TGAAAAGAACTGTGCGAAATTC | | |
| STAP1-407R25 | | CACTTTCTGTGTTCTCTGTCTTCAG | | |
| STAP1-355P27 | | CCTTGTTTTGCCGAAAGAGGAAGTACA | | |
| | | | | |
| CD24_996_U19 | | GCCAGGGCAATGATGAATG | | |
| CD24_1069_L26 | | CTCAATATGGATAATCAAGAGTTGCT | | |
| CD24_1019_P24 | | TCTACCCCCAGATCCAAGCAGCCT | | |
| | | | | |
| TOP1_1679_U22 | | CCCTGTACTTCATCGACAAGC | | |
| TOP1_1762_L18 | | AGCAGCAGCCCACAGTGT | | |
| TOP1_1708_P26 | | AGAGCAGGCAATGAAAAGGAGGAAG | | |

**Table 12 C**

| **10-gene sign ature** | | | | |
|---|---|---|---|---|
| **Gene symbol** | **Upper primer Sequence** | **Probe Sequence** | | **Lower primer Sequence** |
| TULP4 | GAAGAGTGTGTGTCTATGTGCATTTAAA | CAAGTTGCTCCATCTGATTCTTAAATT | | CACATTCACACGGGAAGACAGGCTCA |
| AA741300 | Not available | | | |
| ESCO1 | CTAAACGGCAGCACAAAAGGA | CATGTCTTATGGCTAACACGTTTCTT | | TGCAAACCAACAGACTCAGCAAACAAGG |
| EIF5B | CAGCCAAGGCATCAAGATCA | GAGCGCCATTGACAAGCAAT | | TCATCCTTGGTGCTGTCTTCGCTCTTGTT |
| ACTR2 | CATTCAACTCCAGGACATGGAA | TCCCCAAGACACCAGAATAAAACT | | AGGCCTCTCTCTGCCCTTTGACTGGA |
| WNK1 | GCATGCTTGAGATGGCTACATC | TGGTCACGCGACGGTAGAT | | TCCTTACTCGGAGTGCCAAAATGCTGC |
| COCH | CCATTTAGGCAAATAAGCACTCCTT | GCCTCAGCAGTGTTTTTAACAAAG | | AAGCCGCTGCCTTCTGGTTACAATTTACA |
| SON | GCTCTGCTCAGCCCTAAAGAAA | TCCTCAATATTGGCAGAAAATCCT | | CCTCCCCCTCCTAAAGAGACACTGCCTG |
| TPR | CTGCCCAAGTCTGTCCAGAAC | CCTGACTGTGGGACAACCTCTT | | ATCAGCAATCCGAGATCGATGGCCT |
| NOG | CACCCGGACACTTGATCGAT | GTTCATTGAAAACCCTCGCTAGA | | ACCGCCTCCAACCAGTTCCACCAC |
| | | | | |

| **Gene Symbol** | **Primer sequences** | **Gene symbol** | **Primer sequences** | |
|---|---|---|---|---|
| TULP4-F1 | GAAGAGTGTGTGTCTATGTGCATTTAAA | COCH-F1 | CCATTTAGGCAAATAAGCACTCCTT | |
| TULP4-R1 | CAAGTTGCTCCATCTGATTCTTAAATT | COCH-R1 | GCCTCAGCAGTGTTTTTAACAAAG | |
| TULP4-Pro1 | CACATTCACACGGGAAGACAGGCTCA | COCH-Pro1 | AAGCCGCTGCCTTCTGGTTACAATTTACA | |
| | | | | |
| ESCO1-F1 | CTAAACGGCAGCACAAAAGGA | SON-F1 | GCTCTGCTCAGCCCTAAAGAAA | |
| ESCO1-R1 | CATGTCTTATGGCTAACACGTTTCTT | SON-R1 | TCCTCAATATTGGCAGAAAATCCT | |
| ESCO1-Pro2 | TGCAAACCAACAGACTCAGCAAACAAGG | SON-Pro1 | CCTCCCCCTCCTAAAGAGACACTGCCTG | |
| | | | | |
| NOG-F1 | CACCCGGACACTTGATCGAT | EIF5B-F1 | CAGCCAAGGCATCAAGATCA | |
| NOG-R1 | GTTCATTGAAAACCCTCGCTAGA | EIF5B-R1 | GAGCGCCATTGACAAGCAAT | |
| NOG-Pro1 | ACCGCCTCCAACCAGTTCCACCAC | EIF5B-Pro1 | TCATCCTTGGTGCTGTCTTCGCTCTTGTT | |
| | | | | |
| WNK1-F1 | GCATGCTTGAGATGGCTACATC | ACTR2-F1 | CATTCAACTCCAGGACATGGAA | |
| WNK1-R1 | TGGTCACGCGACGGTAGAT | ACTR2-R1 | TCCCCAAGACACCAGAATAAAACT | |
| WNK1-Pro1 | TCCTTACTCGGAGTGCCAAAATGCTGC | ACTR2-Pro1 | AGGCCTCTCTCTGCCCTTTGACTGGA | |
| | | | | |
| | | TPR-F1 | CTGCCCAAGTCTGTCCAGAAC | |
| | | TPR-R1 | CCTGACTGTGGGACAACCTCTT | |
| | | TPR-Pro1 | ATCAGCAATCCGAGATCGATGGCCT | |

## Claims

1. A method of diagnosing Diabetes Mellitus in a patient comprising the steps of:
a. providing a test sample taken from a patient;
b. measuring the gene expression profile of a gene signature comprising the TOP1, CD24 and STAP1 genes;
c. comparing said gene expression profile with a diagnostic gene expression profile of said gene signature;
d. determining a diabetic disease state in said patient based at least in part upon a substantial match between said gene expression profile and said diagnostic gene expression profile;
e. displaying said determination to a medical professional.

2. The method of claim 1, wherein said determining step is executed by a computer system, said computer system running one or more algorithms selected from the group consisting of Linear combination of gene expression signals, Linear regression model, Logistic regression model, Linear discrimination analysis (LDA) model, The nearest neighbor model and the Prediction Analysis of Microarrays (PAM).

3. The method of claim 2, wherein said determining step further comprises an analysis of the patient's metabolic disease profile.

4. The method of claim 1, wherein said gene signature further comprises one or more genes selected from the genes listed in TABLES 1 or 6.

5. The method of claim 1, wherein said diabetic disease state is a pre-diabetic disease state or a Type 2 Diabetes disease state.

6. The method of claim 1, wherein said test sample is a blood sample.

7. The method of claim 1, wherein said test sample comprises PBMCs or CD11c⁺ or CD11b⁺ or Emr⁺ or [CD11b⁺ CD11c⁺] or [Emr⁺ CD11b⁺] or [Emr⁺ CD11c⁺] or [Emr⁺ CD11b⁺ CD11c⁺] cells or CD14⁺ monocytes.

8. The method of claim 1, wherein said measuring step involves real-time PCR or an immunochemical assay or specific oligonucleotide hybridization.

9. A method of diagnosing a change in the diabetic disease state of a patient comprising the steps of:
a. providing a first test sample taken from a patient at a first time point;
b. measuring a first expression profile of a gene signature in said first test sample, said gene signature comprising the TOP1, CD24 and STAP1 genes;
c. providing a second test sample taken from said patient at a second time point;
d. measuring a second expression profile of said gene signature in said second test sample;
e. comparing said first expression profile with said second expression profile;
f. determining a change in the diabetic disease state in said patient based at least in part upon a substantial difference between said first gene expression profile and said second gene expression profile; and
g. displaying said determination to a medical professional.

10. The method of claim 9, wherein said determining step is executed by a computer system, said computer system running one or more algorithms selected from the group consisting of Linear combination of gene expression signals, Linear regression model, Logistic regression model, Linear discrimination analysis (LDA) model, The nearest neighbor model and the Prediction Analysis of Microarrays (PAM).

11. The method of claim 9, wherein said determining step further comprises an analysis of the patient's metabolic disease profile.

12. The method of claim 9, wherein the time period between said first time point and said second time point is from 0 to 2 years or from ¼ to 2 years or from ½ to 2 years or from 2 to 5 years, or from 5 to 10 years or more.

13. The method of claim 9, wherein said diabetic disease state is a pre-diabetic disease state or a Type 2 Diabetes disease state.

14. The method of claim 9, wherein said first and second test samples are blood samples.

15. The method of claim 9, wherein said measuring step involves real-time PCR, an immunochemical assay or specific oligonucleotide hybridization.

16. A kit for assessing a patient's susceptibility to Diabetes in which the assessment is made with a test apparatus, the kit comprising:
a. reagents for collecting a test sample from a patient; and
b. reagents for measuring the gene expression profile of a gene signature comprising the TOP1, CD24 and STAP1 genes in a patient's test sample, wherein the reagents are for: (i) real-time PCR, and wherein said reagents comprise primers for the amplification and detection of the gene expression profile, or (ii) an immunochemical assay, wherein said reagents comprise antibodies for the detection of the TOP1, CD24 and STAP1 proteins; and packaging therefor.

17. Use of a kit in assessing a patient's susceptibility to Diabetes according to the method of claim 1, in which the assessment is made with a test apparatus, the kit comprising:
a. reagents for collecting a test sample from a patient; and
b. reagents for measuring the gene expression profile of a gene signature comprising the TOP1, CD24 and STAP1 genes in a patient's test sample; and packaging therefor.

18. The kit of claim 16 or the use of claim 17, wherein said reagents for collecting a test sample are reagents for collecting a blood sample.

19. The use of claim 17, wherein said reagents for measuring the expression profile of a gene signature are reagents for real-time PCR or an immunochemical assay or specific oligonucleotide hybridization.

## Patentansprüche

1. Verfahren zur Diagnose von Diabetes mellitus bei einem Patienten, wobei man in den Verfahrensschritten:
a. eine einem Patienten entnommene Testprobe bereitstellt;
b. das Genexpressionsprofil einer die Gene für TOP1, CD24 und STAP1 umfassenden Gensignatur misst;
c. das Genexpressionsprofil mit einem diagnostischen Genexpressionsprofil der Gensignatur vergleicht;
d. einen diabetischen Krankheitszustand bei dem Patienten wenigstens teilweise auf der Basis einer weitgehenden Übereinstimmung zwischen dem Genexpressionsprofil und dem diagnostischen Genexpressionsprofil bestimmt;
e. die Bestimmung einem Berufsmediziner präsentiert.

2. Verfahren nach Anspruch 1, wobei der Bestimmungs-schritt mit einem Computersystem ausgeführt wird, wobei auf dem Computersystem ein oder mehrere aus der aus linearer Kombination von Genexpressions-signalen, linearem Regressionsmodell, logistischem Regressionsmodell, LDA(Linear Discrimination Analysis)-Modell, dem Nearest-Neighbor-Modell und PAM (Prediction Analysis of Microarrays) bestehenden Gruppe ausgewählte Algorithmen laufen.

3. Verfahren nach Anspruch 2, wobei der Bestimmungsschritt ferner eine Analyse des metabolischen Krankheitsprofils des Patienten umfasst.

4. Verfahren nach Anspruch 1, wobei die Gensignatur ferner ein oder mehrere aus den in TABELLE 1 oder 6 aufgeführten Genen ausgewählte Gene umfasst.

5. Verfahren nach Anspruch 1, wobei es sich bei dem diabetischen Krankheitszustand um einen prädiabetischen Krankheitszustand oder einen Typ-2-Diabetes-Krankheitszustand handelt.

6. Verfahren nach Anspruch 1, wobei es sich bei der Testprobe um eine Blutprobe handelt.

7. Verfahren nach Anspruch 1, wobei die Testprobe PBMC oder CD11c⁺- oder CD11b⁺- oder Emr⁺- oder [CD11b⁺ CD11c⁺]- oder [Emr⁺ CD11b⁺] - oder [Emr⁺ CD11c⁺] - oder [Emr⁺ CD11b⁺ CD11c⁺] -Zellen oder CD14⁺-Monozyten umfasst.

8. Verfahren nach Anspruch 1, wobei der Messschritt Echtzeit-PCR oder ein immunchemisches Testverfahren oder spezifische Oligonukleotidhybridisierung beinhaltet.

9. Verfahren zur Diagnose einer Änderung des diabetischen Krankheitszustands eines Patienten, wobei man in den Verfahrensschritten:
a. eine einem Patienten zu einem ersten Zeitpunkt entnommene erste Testprobe bereitstellt;
b. ein erstes Expressionsprofil einer Gensignatur in der ersten Testprobe misst, wobei die Gensignatur die Gene für TOP1, CD24 und STAP1 umfasst;
c. eine dem Patienten zu einem zweiten Zeitpunkt entnommene zweite Testprobe bereitstellt;
d. ein zweites Expressionsprofil der Gensignatur in der zweiten Testprobe misst;
e. das erste Expressionsprofil mit dem zweiten Expressionsprofil vergleicht;
f. eine Änderung des diabetischen Krankheitszustands bei dem Patienten wenigstens teilweise auf der Basis eines wesentlichen Unterschieds zwischen dem ersten Genexpressionsprofil und dem zweiten Genexpressionsprofil bestimmt; und g. die Bestimmung einem Berufsmediziner präsentiert.

10. Verfahren nach Anspruch 9, wobei der Bestimmungsschritt mit einem Computersystem ausgeführt wird, wobei auf dem Computersystem ein oder mehrere aus der aus linearer Kombination von Genexpressionssignalen, linearem Regressionsmodell, logistischem Regressionsmodell, LDA(Linear Discrimination Analysis)-Modell, dem Nearest-Neighbor-Modell und PAM (Prediction Analysis of Microarrays) bestehenden Gruppe ausgewählte Algorithmen laufen.

11. Verfahren nach Anspruch 9, wobei der Bestimmungsschritt ferner eine Analyse des metabolischen Krankheitsprofils des Patienten umfasst.

12. Verfahren nach Anspruch 9, wobei der Zeitraum zwischen dem ersten Zeitpunkt und dem zweiten Zeitpunkt 0 bis 2 Jahre oder ¼ bis 2 Jahre oder ½ bis 2 Jahre oder 2 bis 5 Jahre oder 5 bis 10 Jahre oder mehr beträgt.

13. Verfahren nach Anspruch 9, wobei es sich bei dem diabetischen Krankheitszustand um einen prädiabetischen Krankheitszustand oder einen Typ-2-Diabetes-Krankheitszustand handelt.

14. Verfahren nach Anspruch 9, wobei es sich bei der ersten und zweiten Testprobe um Blutproben handelt.

15. Verfahren nach Anspruch 9, wobei der Messschritt Echtzeit-PCR, ein immunchemisches Testverfahren oder spezifische Oligonukleotidhybridisierung beinhaltet.

16. Kit zur Beurteilung der Anfälligkeit eines Patienten für Diabetes, wobei die Beurteilung mit einer Testvorrichtung erfolgt, wobei das Kit:
a. Reagentien zum Sammeln einer Testprobe von einem Patienten; und
b. Reagentien zum Messen des Genexpressionsprofils einer die Gene für TOP1, CD24 und STAP1 umfassenden Gensignatur in einer Patiententestprobe, wobei die Reagentien für: (i) Echtzeit-PCR, wobei die Reagentien Primer für die Amplifikation und den Nachweis des Genexpressionsprofils umfassen, oder (ii) ein immunchemisches Testverfahren, wobei die Reagentien Antikörper für den Nachweis der Proteine TOP1, CD24 und STAP1 umfassen, vorgesehen sind; und Verpackungsmaterial dafür umfasst.

17. Verwendung eines Kits bei der Beurteilung der Anfälligkeit eines Patienten für Diabetes gemäß dem Verfahren nach Anspruch 1, wobei die Beurteilung mit einer Testvorrichtung erfolgt, wobei das Kit:
a. Reagentien zum Sammeln einer Testprobe von einem Patienten; und
b. Reagentien zum Messen des Genexpressionsprofils einer die Gene für TOP1, CD24 und STAP1 umfassenden Gensignatur in einer Patiententestprobe; und Verpackungsmaterial dafür umfasst.

18. Kit nach Anspruch 16 oder Verwendung nach Anspruch 17, wobei es sich bei den Reagentien zum Sammeln einer Testprobe um Reagentien zum Sammeln einer Blutprobe handelt.

19. Verwendung nach Anspruch 17, wobei es sich bei den Reagentien zum Messen des Expressionsprofils einer Gensignatur um Reagentien für Echtzeit-PCR oder ein immunchemisches Testverfahren oder spezifische Oligonukleotidhybridisierung handelt.

## Revendications

1. Procédé de diagnostic du diabète sucré chez un patient comprenant les étapes de :
a. fourniture d'un échantillon d'essai prélevé sur un patient ;
b. mesure du profil d'expression génique d'une signature génique comprenant les gènes TOP1, CD24 et STAP1 ;
c. comparaison dudit profil d'expression génique à un profil d'expression génique diagnostique de ladite signature génique ;
d. détermination d'un état pathologique diabétique chez ledit patient sur la base, au moins en partie, d'une correspondance substantielle entre ledit profil d'expression génique et ledit profil d'expression génique diagnostique ;
e. Présentation de ladite détermination à un professionnel médical.

2. Procédé de la revendication 1, dans lequel ladite étape de détermination est exécutée par un système informatique, ledit système informatique exécutant un ou plusieurs algorithmes choisis dans le groupe constitué d'une combinaison linéaire de signaux d'expression génique, un modèle de régression linéaire, un modèle de régression logistique, un modèle d'analyse discriminante linéaire (LDA), le modèle de plus proche voisin et la méthode PAM (Prediction Analysis of Microarrays).

3. Procédé de la revendication 2, dans lequel ladite étape de détermination comprend en outre une analyse du profil de maladie métabolique du patient.

4. Procédé de la revendication 1, dans lequel ladite signature génique comprend en outre un ou plusieurs gènes choisis parmi les gènes répertoriés dans les TABLEAUX 1 ou 6.

5. Procédé de la revendication 1, dans lequel ledit état pathologique diabétique est un état pathologique pré-diabétique ou un état pathologique diabétique de type 2.

6. Procédé de la revendication 1, dans lequel ledit échantillon d'essai est un échantillon de sang.

7. Procédé de la revendication 1, dans lequel ledit échantillon d'essai comprend des PBMC ou des cellules CD11c⁺ ou CD11b⁺ ou Emr⁺ ou [CD11b⁺ CD11c⁺] ou [Emr⁺ CD11b⁺] ou [Emr⁺ CD11c⁺] ou [Emr⁺ CD11b⁺ CD11c⁺] ou des monocytes CD14⁺.

8. Procédé de la revendication 1, dans lequel ladite étape de mesure met en oeuvre la PCR en temps réel ou un dosage immunochimique ou l'hybridation d'oligonucléotide spécifique.

9. Procédé de diagnostic d'un changement de l'état pathologique diabétique d'un patient comprenant les étapes de :
a. fourniture d'un premier échantillon d'essai prélevé sur un patient à un premier temps ;
b. mesure d'un premier profil d'expression d'une signature génique dans ledit premier échantillon d'essai, ladite signature génique comprenant les gènes TOP1, CD24 et STAP1 ;
c. fourniture d'un deuxième échantillon d'essai prélevé sur ledit patient à un deuxième temps ;
d. mesure d'un deuxième profil d'expression de ladite signature génique dans ledit deuxième échantillon d'essai ;
e. comparaison dudit premier profil d'expression audit deuxième profil d'expression ;
f. détermination d'un changement de l'état pathologique diabétique chez ledit patient sur la base, au moins en partie, d'une différence substantielle entre ledit premier profil d'expression génique et ledit deuxième profil d'expression génique ; et
g. présentation de ladite détermination à un professionnel médical.

10. Procédé de la revendication 9, dans lequel ladite étape de détermination est exécutée par un système informatique, ledit système informatique exécutant un ou plusieurs algorithmes choisis dans le groupe constitué d'une combinaison linéaire de signaux d'expression génique, un modèle de régression linéaire, un modèle de régression logistique, un modèle d'analyse discriminante linéaire (LDA), le modèle de plus proche voisin et la méthode PAM (Prediction Analysis of Microarrays).

11. Procédé de la revendication 9, dans lequel ladite étape de détermination comprend en outre une analyse du profil de maladie métabolique du patient.

12. Procédé de la revendication 9, dans lequel la durée entre ledit premier temps et ledit deuxième temps est de 0 à 2 ans ou de ¼ à 2 ans ou de ½ à 2 ans ou de 2 à 5 ans, ou de 5 à 10 ans ou plus.

13. Procédé de la revendication 9, dans lequel ledit état pathologique diabétique est un état pathologique pré-diabétique ou un état pathologique diabétique de type 2.

14. Procédé de la revendication 9, dans lequel lesdits premier et deuxième échantillons d'essai sont des échantillons de sang.

15. Procédé de la revendication 9, dans lequel ladite étape de mesure met en oeuvre la PCR en temps réel, un dosage immunochimique ou l'hybridation d'oligonucléotide spécifique.

16. Kit pour évaluer la susceptibilité d'un patient au diabète, l'évaluation étant effectuée avec un appareil d'essai, le kit comprenant :
a. des réactifs pour collecter un échantillon d'essai à partir d'un patient ; et
b. des réactifs pour mesurer le profil d'expression génique d'une signature génique comprenant les gènes TOP1, CD24 et STAP1 dans un échantillon d'essai d'un patient, les réactifs étant pour : (i) la PCR en temps réel, auquel cas lesdits réactifs comprennent des amorces pour l'amplification et la détection du profil d'expression génique, ou (ii) un dosage immunochimique, auquel cas lesdits réactifs comprennent des anticorps pour la détection des protéines TOP1, CD24 et STAP1 ; et un emballage pour ceux-ci.

17. Utilisation d'un kit dans l'évaluation de la susceptibilité d'un patient au diabète selon le procédé de la revendication 1, l'évaluation étant effectuée avec un appareil d'essai, le kit comprenant :
a. des réactifs pour collecter un échantillon d'essai à partir d'un patient ; et
b. des réactifs pour mesurer le profil d'expression génique d'une signature génique comprenant les gènes TOP1, CD24 et STAP1 dans un échantillon d'essai d'un patient ; et un emballage pour ceux-ci.

18. Kit de la revendication 16 ou utilisation de la revendication 17, lesdits réactifs pour collecter un échantillon d'essai étant des réactifs pour collecter un échantillon de sang.

19. Utilisation de la revendication 17, lesdits réactifs pour mesurer le profil d'expression d'une signature génique étant des réactifs pour la PCR en temps réel ou un essai immunochimique ou l'hybridation d'oligonucléotide spécifique.
